Europäisches Patentamt
European Patent Office
Office européen des brevets

(19)

(11) Publication number: **0 268 871**
**A1**

(12)

# EUROPEAN PATENT APPLICATION

(21) Application number: 87116008.1

(22) Date of filing: 30.10.87

(51) Int. Cl.4: C07D 219/10 , C07D 221/16 , C07D 221/22 , C07D 471/18 , C07D 495/04 , A61K 31/645 , A61K 31/47 , //(C07D471/18,221:00,221:00,221:00),(C07D495/04,335:00,221:00),(C07D495/04,333:00,221:00)

(30) Priority: 31.10.86 JP 261579/86
31.10.86 JP 261580/86
05.11.86 JP 262983/86
29.12.86 JP 310930/86
29.12.86 JP 310931/86
30.03.87 JP 78483/87
15.05.87 JP 119685/87
15.05.87 JP 119686/87
15.05.87 JP 119687/87

(43) Date of publication of application:
01.06.88 Bulletin 88/22

(84) Designated Contracting States:
AT BE CH DE ES FR GB GR IT LI LU NL SE

(71) Applicant: SUMITOMO PHARMACEUTICALS COMPANY, LIMITED
40, Dosho-machi 2-chome
Higashi-ku Osaka(JP)

(72) Inventor: Kawakami, Hajime
6-32-702, Fukazucho
Nishinomiya-shi(JP)
Inventor: Ohuchi, Renzo
10-3-326, Sonehigashinocho-2-chome
Toyonaka-shi(JP)
Inventor: Kitano, Masahumi
Sumitomo Kagaku Yutokuryo 29-7
Oike-2-chome
Ibaraki-shi(JP)
Inventor: Ono, Keiichi
10-4, Momoyamadai-3-cho
Sakai-shi(JP)

(74) Representative: Vossius & Partner
Siebertstrasse 4 P.O. Box 86 07 67
D-8000 München 86(DE)

(54) **Quinoline derivatives.**

(57) Quinoline derivatives of the formula,

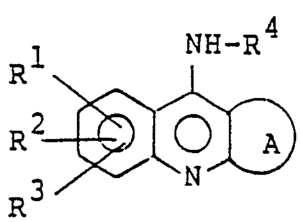

(I)

EP 0 268 871 A1

wherein $R^1$, $R^2$, $R^3$ and $R^4$ are each hydrogen or one of a specific range of substituents,

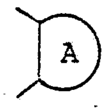

is one of a specific range of cyclic groups, and are useful as pharmaceuticals such as anti-senile dementia. They may be prepared by synthetic routes analogous to those described in the literature for preparing known compounds.

# QUINOLINE DERIVATIVES

## BACKGROUND OF THE INVENTION

### 1. FIELD OF THE INVENTION

The present invention relates to quinoline derivatives and pharmaceutically acceptable acid addition salts thereof, processes for preparation thereof and pharmaceutical use thereof. More particularly, it relates to novel quinoline derivatives which increase acetylchorine level in the brain by inhibiting an action of acetylcholinesterase and therefore are useful for treatment of various memory dysfunctions characterized by decreased function, such as Alzheimer's disease and senile dementia of Alzheimer's type.

### 2. RELATED ART STATEMENT

It has been known that acetylcholine level decreases in the brain of patients suffering from Alzheimer's disease [In the Aging Brain, Berlin, p. 140 (1982)]. It has been examined whether or not physostigmine, which is acetylcholinesterease inhibitor, is efficacious for senile dementia [Neurology, 8, p. 397 (1978)]. Additionally it has been reported that 9-amino-1,2,3,4-tetrahydroacridinol derivatives [Japanese Patent KOKAI (Laid-Open) No. 148154/86] and benzo [c]-1,5-naphthyridine derivatives [Japanese Patent KOKAI (Laid-Open) No. 212585/86] have inhibitory activity against acetylcholinesterase.

It has been reported that aminoacridine derivatives exhibit analgesic, sedative, and antidepressant activities (U.S. Patent No. 3,232,945). It has been known that a combination of tacrine (9-amino-1,2,3,4-tetrahydroacridine) which inhibits an action of acetylcholinesterase with lecithin is useful for treating Alzheimer's disease through intravenous injection thereof [Neurobiology Aging, 4, p. 139 (1983)].

It has been reported that acetylcholinesterease inhibitor is useful for treatment of various memory dysfunctions characterized by decreased cholinergic function because it increases acetylcholine level in the brain. However, acetylcholinesterase inhibitor without adverse side effects has not been found yet.

## SUMMARY OF THE INVENTION

Accordingly, the primary object of the present invention is to provide novel quinoline derivatives having excellent inhibitory activity against acetylcholinesterase and also exhibiting strong effects on amnestic models in the several animal without adverse side effects and therefore useful for treatment of senile dementia.

Another object of the present invention is to provide processes for production of the novel quinoline derivatives.

A further object of the present invention is to provide a pharmaceutical use of the novel quinoline derivatives.

These and other objects and advantages of the present invention will become clear from the following description.

The present invention pertains to novel quinoline derivatives represented by the formula,

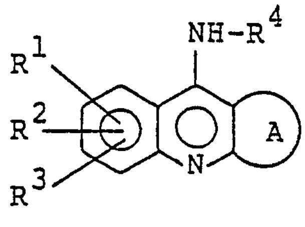

(I)

and pharmaceutically acceptable acid addition salt thereof wherein $R^1$, $R^2$, $R^3$, $R^4$, and

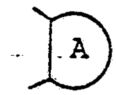

are defined as the following (1) or (2);

(1) 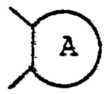 is 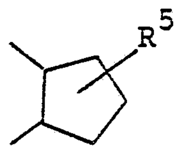, 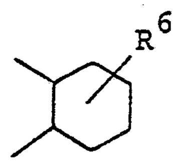, or

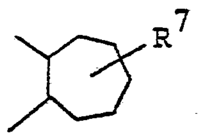

wherein $R^5$, $R^6$ and $R^7$ are each independently hydrogen or lower alkyl; $R^1$ is lower alkyl, halogen, trifluoromethyl, nitro, amino, hydroxy, lower alkylamino, lower alkanoylamino, lower alkylthio, lower alkoxy, or lower alkoxymethyl; $R^2$ and $R^3$ are each independently hydrogen, halogen, lower alkyl, lower alkoxy, lower cycloalkyl, phenyl, or substituted phenyl; and $R^4$ is hydrogen; provided that when $R^1$ is lower alkyl, lower alkoxy, chlorine, bromine, or iodine and $R^2$ and $R^3$ are each hydrogen,

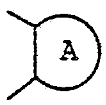 is 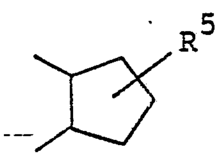 or 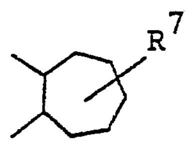

wherein $R^5$ and $R^7$ are each independently lower alkyl;

(2) 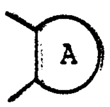 is 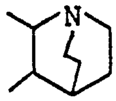 , 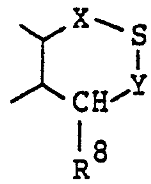 ,

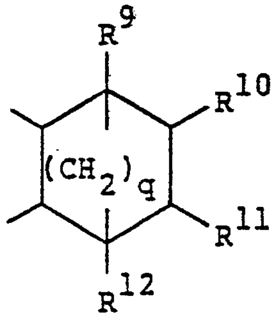 , 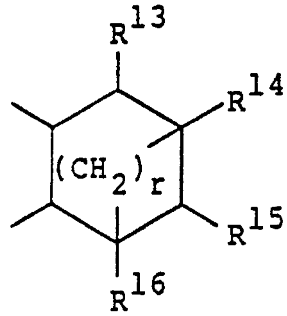 , 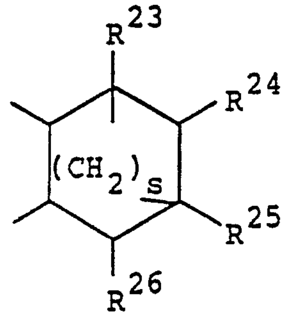 ,

or 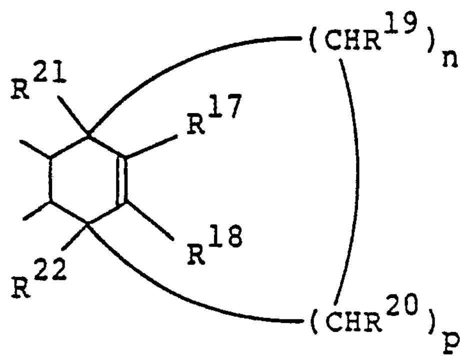

wherein n, q, r, and s are each independently 1 or 2, p is 0 or 1, X and Y are each independently bond, alkylene, or substituted alkylene, and $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, and $R^{26}$ are each independently hydrogen or lower alkyl, provided that the total of carbon atoms of X and Y is an integer 1 to 3; $R^1$, $R^2$, and $R^3$ are each independently hydrogen, halogen, trifluoromethyl, nitro, amino, hydroxy, lower alkyl, lower alkoxy, lower alkylamino, lower alkanoylamino, lower cycloalkyl, lower alkylthio, lower alkoxymethyl, phenyl, or substituted phenyl; and $R^4$ is hydrogen, lower alkyl, aralkyl, or diaralkyl.

## DETAILED DESCRIPTION OF THE INVENTION

In the significances as defined above, the term "substituted phenyl" means phenyl having halogen, lower alkyl, or lower alkoxy substituents. The term "alkylene" includes methylene, ethylene, and trimethylene. The term "halogen" includes fluorine, chlorine, bromine, and iodine. The term "substituted alkylene" means alkylene having methyl or ethyl substituents. The term "lower alkyl" means a straight or branched chain alkyl group having 1 to 4 carbon atoms (e.g. methyl, ethyl, n-propyl, isopropyl, n-butyl, sec-butyl etc.). The term "lower alkoxy" means a straight or branched chain alkoxy group having 1 to 4 carbon atoms (e.g. methoxy, ethoxy, n-propoxy, isopropoxy, n-butoxy, sec-butoxy etc.). The term "lower alkylthio" means a straight or branched chain alkylthio group having 1 to 4 carbon atoms (e.g. methylthio, ethylthio, n-propylthio, isopropylthio, n-butylthio, sec-butylthio etc.). The term "lower alkoxymethyl" means a straight or branched chain alkoxymethyl group having 2 to 5 carbon atoms (e.g. methoxymethyl, ethoxymethyl, n-propoxymethyl, isopropoxymethyl, n-butoxymethyl, sec-butoxymethyl etc.). The term "lower alkylamino" means a straight or branched chain alkylamino group having 1 to 4 carbon atoms (e.g. methylamino, ethylamino, n-propylamino, isopropylamino, n-butylamino, sec-butylamino etc.). The term "lower alkanoylamino" means a straight. or branched chain alkanoylamino group having 2 to 5 carbon atoms (e.g. acetylamino, n-propionylamino. isopropionylamino, n-butyrylamino, iso-butyrylamino, sec-butyrylamino etc.).

The term "lower cycloalkyl" means cycloalkyl or lower alkyl substituted cycloalkyl having 3 to 10 carbon atoms including those of the substituent(s) (e.g.cyclopropyl, cyclopentyl, cyclohexyl, cycloheptyl etc.). The term "aralkyl" means phenylalkyl or substituted phenylalkyl having 7 to 13 carbon atoms including those of the substituents) e.g. benzyl, phenetyl, 2-(4-methoxyphenyl)ethyl, 2-(4-methylphenyl)ethyl, 2-(4-fluorophenyl)ethyl, 2-naphtylmethyl, 3-phenylpropyl, 4-phenylbutyl, 4-(4-fluorophenyl)butyl etc.]. The term "diaralkyl" means diphenylalkyl or substituted diphenylalkyl having 13 to 20 carbon atoms including those of the substituent(s) [e.g. 4,4-diphenylbutyl, 4,4-bis(4-fluorophenyl)butyl, 4,4-bis(4-methoxyphenyl)butyl, 4,4-bis(4-methylphenyl)butyl, 3,3-diphenylpropyl, 2,2-diphenylethyl etc.].

The quinoline derivatives (I) may be in the form of their pharmaceutically acceptable acid addition salts. Acids useful for preparing the pharmaceutically acceptable acid addition salts include inorganic acids such as hydrochloric acid, hydrobromic acid, sulfuric acid, nitric acid, phosphoric acid, perchloric acid; and organic acids such as tartaric acid, succinic acid, maleic acid, fumaric acid.

The quinoline derivatives (I) inhibit the action of acetylcholinesterase and show an excellent effect on the amnestic model in several animals without adverse side effects.

Among the quinoline derivatives (I) of the present invention, the following compounds (1) - (5) and pharmaceutically acceptable acid addition salts thereof are preferable;

(1) those of the formula (I) wherein

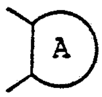 is 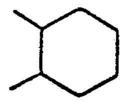 ,

$R^1$ is fluorine, and $R^2$ and $R^3$ are each independently hydrogen or fluorine, more preferably, $R^1$ is fluorine, and $R^2$ and $R^3$ are hydrogen:

(2) those of the formula (I) wherein

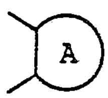 is 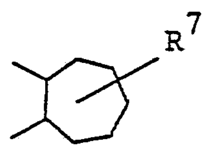 ,

$R^7$ is hydrogen, $R^1$ is fluorine, $R^2$ is hydrogen or fluorine, and $R^3$ is hydrogen:

(3) those of the formula (I) wherein

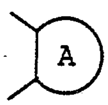 is 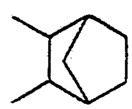 ,

$R^1$, $R^2$, and $R^3$ are each independently hydrogen, halogen, lower alkyl, or lower alkoxy, and $R^4$ is hydrogen, more preferably, $R^1$ and $R^2$ are each fluorine, and $R^3$ and $R^4$ are each hydrogen:

(4) those of the formula (I) wherein

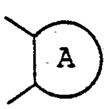 is 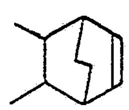 ,

$R^1$, $R^2$, and $R^3$ are each independently hydrogen or halogen, and $R^4$ is hydrogen, more preferably, $R^1$, $R^2$, and $R^3$ each hydrogen, and $R^4$ is hydrogen:

(5) those of the formula (I) wherein

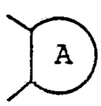 is 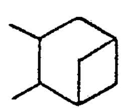 ,

$R^1$ is hydrogen, halogen, or lower alkyl, and $R^2$, $R^3$, and $R^4$ are each hydrogen, more preferably, $R^1$, $R^2$, .$R^3$, and $R^4$ are each hydrogen; and $R^1$ is fluorine, and $R^2$, $R^3$, and $R^4$ are each hydrogen.

According to the present invention, the quinoline derivatives (1) are prepared preferably by the following synthetic route.

This route is almost the same as described in Tetrahedron Letters, p. 1277, (1963) and represented by the following reaction scheme;

Reaction Scheme

2-Aminobenzonitrile Derivatives (II) + Cycloalkanone Derivatives (III) - (XI)

Lewis acid or polyphosphoric acid

Quinoline Derivatives (I)

The preparation of the compound (I) or their pharmaceutically acceptable acid addition salts can thus be conducted by

(a) reacting a compound (II) of the formula,

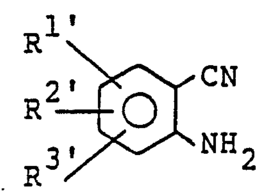

(II)

wherein $R^1$ is lower alkyl, lower alkoxy, chlorine, bromine, or iodine and $R^{2'}$ and $R^{3'}$ are each hydrogen, with a compound (III) or (V) of the formula,

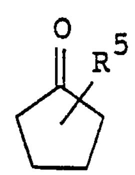

(III)

or

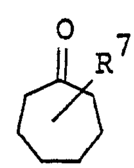

(V)

wherein $R^5$ and $R^7$ are each lower alkyl, in the presence of Lewis acid or dehydrating-condensing agent;

(b) reacting a compound (II) of the formula,

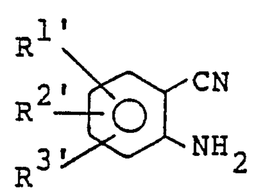

(II)

wherein $R^{1'}$ is lower alkyl, halogen, trifluoromethyl, nitro, hydroxy, lower alkanoylamino, lower alkylthio, lower alkoxy, or lower alkoxymethyl; $R^{2'}$ and $R^{3'}$ are each independently hydrogen, halogen, lower alkyl, lower alkoxy, lower cycloalkyl, phenyl, or substituted phenyl; provided that the case is excluded where $R^1$ is lower alkyl, lower alkoxy, chlorine, bromine, or iodine and $R^2$ and $R^3$ are each hydrogen, with a compound (III), (IV) or (V) of the formula,

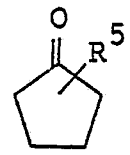
(III)

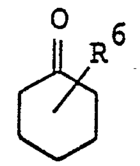
(IV)

or

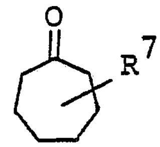
(V)

wherein $R^5$, $R^6$ and $R^7$ are each independently hydrogen or lower alkyl, in the presence of Lewis acid or dehydrating-condensing agent; or

(c) reacting a compound (II) of the formula,

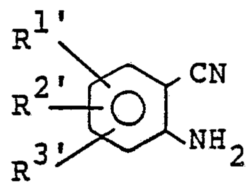
(II)

$R^{1'}$, $R^{2'}$ and $R^{3'}$ are each independently hydrogen, halogen, trifluoromethyl, nitro, hydroxy, lower alkyl, lower alkoxy, lower alkanoylamino, lower cycloalkyl, lower alkylthio, lower alkoxymethyl, phenyl, or substituted phenyl, with a compound (VI), (VII), (VIII), (IX), (X) or (XI) of the formula,

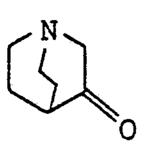
(VI)

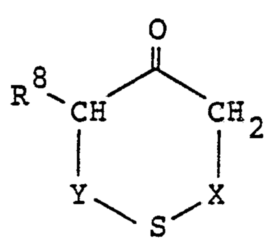
(VII)

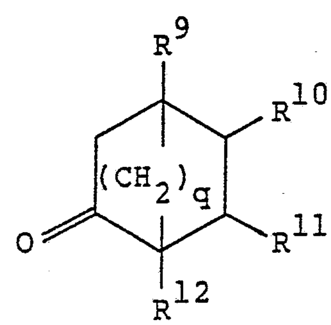
(VIII)

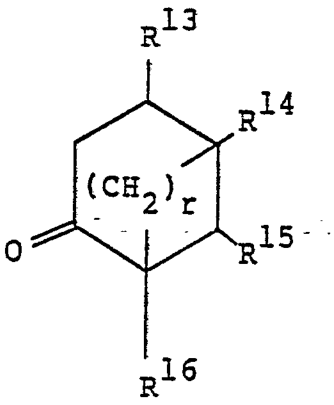

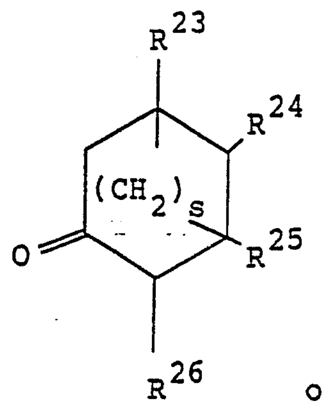

or

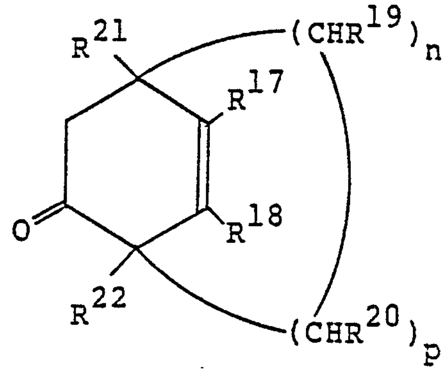

(IX) (X) (XI)

wherein n, q, r, and s are each independently 1 or 2, p is 0 or 1, X and Y are each independently bond, alkylene, or substituted alkylene, and $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, and $R^{26}$ are each independently hydrogen or lower alkyl, provided that the total of carbon atoms of X and Y is an integer 1 to 3; in the presence of Lewis acid or dehydrating-condensing agent.

The compound (I) thus obtained by the reaction (b) or (c) wherein at least one of $R^1$, $R^2$ and $R^3$ is a nitro group may be subjected to reduction of the nitro group to give the compound (I) having at least one amino group. The thus obtained compound (I) may further be subjected to alkylation or acylation of the amino group to give the compound (I) wherein at least one of $R^1$, $R^2$ and $R^3$ is a lower alkylamino or lower alkanoylamino.

The compound (I) wherein $R^4$ is lower alkyl, aralkyl, or diaralkyl can be preferably obtained by the following method (i) or (ii) mentioned below.

(i) In a case where the compound (I) is used wherein at least one of $R^1$, $R^2$ and $R^3$ is amino, or lower alkylamino, it is firstly subjected to protection of the amino or lower alkylamino, followed by alkylation, aralkylation or diaralkylation of amino group of $R^4$ and thereafter deprotection. The preferred protective groups include tert-butoxycarbonyl, acetyl and benzoyl.

(ii) In a case where the compound (I) is used wherein at least one of $R^1$, $R^2$ and $R^3$ is anitro group, it is directly subjected to alkylation, aralkylation or diaralkylation of amino group of $R^4$, and if necessary further subjected to reduction of the nitro groups. The thus obtained compound (I) wherein at least one of $R^1$, $R^2$ and $R^3$ is a amino group may be subjected to alkylation or acylation of the amino groups.

The compound (I) obtained by the reaction (a), (b) or (c) may be subjected to a salt-forming reaction.

As the Lewis acid used in the reactions of compounds (II), there may be exemplified anhydrous aluminium chloride, anhydrous aluminium bromide, anhydrous zinc chloride, anhydrous zinc bromide, anhydrous zinc iodide, titanium (IV) chloride, anhydrous tin (IV) chloride, or boron trifluoride diethyl etherate etc.

Suitable solvents for the reaction of the compound (II) with the compound (III), (IV), (V), (VI), (VII), (VIII), (IX), (X) or (XI) include N,N-dimethylformide, aromatic hydrocarbon (e.g. xylene, toluene etc.), halogenated hydrocarbon (e.g. chloroform, dichloromethane, 1,2-dichloroethane, 1,2-dibromoethane etc.), nitrobenzene etc. Also this reaction can be carried out without a solvent. The reaction is conducted at a temperature ranging from 30°C to 200°C, preferably from 70°C to 180°C.

When the compound (II) is reacted with the compound (III), (IV), (V) or (VII), the reaction can be preferably effected in a halogenated hydrocarbon solvent, in nitrobenzene or N,N-dimethylformamide in the presence of anhydrous aluminum chloride, anhydrous aluminum bromide, anhydrous zinc chloride, anhydrous zinc bromide or anhydrous zinc iodide at a temperature from 70°C to 150°C.

When the compound (II) is reacted with the compound (VIII), (IX), (X) or (XI), the reaction can be preferably effected in nitrobenzene in the presence of anhydrous zinc chloride, anhydrous zinc bromide or anhydrous zinc iodide at a temperature from 80°C to 150°C.

The salt-forming reaction can be conducted by conventional methods wherein the obtained compound (I) is allowed to contact with the organic acid or inorganic acid in an appropriate solvent such as in alcohols (e.g. methanol, ethanol, isopropylalcohol etc.).

The reduction of the nitro group can be conducted by conventional methods. For example, the reaction may be preferably effected in the presence of iron and hydrochloric acid. Thus, the compound (I) wherein $R^1$, $R^2$ and/or $R^3$ are amino group(s) can be obtained.

The alkylation, aralkylation or diaralkylation of the amino group can be effected according to the conventional method as described in, for example, Shin Jikken Kagaku Koza (New Experimental Chemistry), Vol. 14, p. 1332, "A reaction and synthesis of organic compound", Japanese Chemical Association. Those reactions can be also conducted in the same manner as described in Japanese Patent KOKAI (Laid-Open) No. 148154/86 wherein the reactions are effected in a strong aqueous alkaline solution such as 50% aqueous sodium hydroxide solution, in an organic solvent such as dichloromethane and toluene, or in two phase-reaction system containing a phase-transfer catalyst such as tetrabutylammonium hydrogen sulfate.

The acylation of the amino group can be conducted by conventional methods. For example, the reaction may be preferably effected by reacting the compound (I) carring amino groups with acylhalide (e.g. acetylchloride, n-propionylchloride, iso-butyrylbromide etc.) in the presence of tertiary amine (e.g. triethyl amine, tributyl amine etc.) [Shin Jikken Kagaku Koza (New Experimental Chemistry), Vol. 14, p. 1134, "A reaction and synthesis of organic compound II", Japanese Chemical Association].

The method of deprotection may vary depending on a kind of protective group. There may be exemplified a method wherein the deprotection is conducted in the presence of acids such as hydrochloric acid [Protecting Groups in Organic Synthesis, p. 251 and 272], one wherein catalytic hydrogenation is conducted in the presence of, for example, palladium-carbon, and one using aqueous alkaline solution such as an aqueous sodium hydroxide solution.

Generally, two isomeric products are obtained by reacting the compound (II) with the compounds (III), (IV), (V), (VI), (VII), (VIII), (IX), (X), or (XI), which have an asymmetric structure and two methylene groups on both sides of the ketone group. They can be separated by general method (e.g. Silica gel column chromatography).

The quinoline derivatives (I) and their acid addition salts can be administrated orally or parenterally in the form of conventional pharmaceutical preparations. For instance, they can be administered orally in the form of conventional solid pharmaceutical preparations such as tablets, capsules, syrups and suspensions. Alternatively, they can be administered parenterally by injection in the form of solutions, emulsions, etc. Also, they may be directly applied to rectum in the form of a suppository. Further, the preparations may contain physiologically acceptable carriers, excipients, activators, binding agents, stabilizers, etc. In the case of injections, physiologically acceptable buffers, solubilizing agents or isotonic agents may be incorporated therein. The daily dosage may vary depending upon the sympton of disease, the age and bodyweight of patient, the administration route, etc., and the normal dosage to a human adult is between 1 mg and 500 mg, preferably between 5 mg and 300 mg dividing in one to several times per day.

Following compounds are embraced and obtainable by the present invention.

Table 1

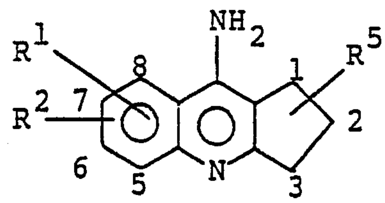

| $R^1$ | $R^2$ | $R^5$ |
|---|---|---|
| F(8) | H | H |
| F(7) | H | H |
| F(6) | H | H |
| F(8) | H | $CH_3$(1) |
| F(6) | F(7) | H |
| F(6) | F(8) | H |
| F(6) | $CH_3$(7) | H |
| F(8) | H | $CH_3$(2) |
| F(8) | H | $CH_3$(3) |
| F(8) | H | $C_2H_5$(3) |
| $CH_3$(8) | H | $CH_3$(2) |
| $CH_3$(8) | H | $CH_3$(3) |
| $OCH_3$(8) | H | $CH_3$(2) |
| $OCH_3$(8) | H | $CH_3$(3) |
| Cl(8) | H | $CH_3$(2) |
| Cl(8) | H | $CH_3$(3) |
| F(5) | H | H |
| F(6) | $CH_3$(7) | $CH_3$(3) |
| F(6) | F(7) | $CH_3$(2) |

- cont'd -

Table 1 (cont'd)

| $R^1$ | $R^2$ | $R^5$ |
|---|---|---|
| F(6) | F(7) | $CH_3$(3) |
| F(6) | F(8) | $CH_3$(2) |
| F(6) | F(8) | $CH_3$(3) |
| $CF_3$(7) | H | H |
| $CF_3$(7) | H | $CH_3$(2) |
| $CF_3$(7) | H | $CH_3$(3) |
| $CF_3$(6) | H | $CH_3$(2) |
| Cl(7) | H | $CH_3$(1) |
| Cl(7) | H | $CH_3$(2) |
| Cl(7) | H | $CH_3$(3) |
| H | H | $CH_3$(2) |
| H | H | $CH_3$(3) |
| $CF_3$(7) | H | $CH_3$(1) |
| H | H | $C_2H_5$(3) |
| $CH_3$(7) | H | $CH_3$(3) |

The numeral in parenthesis indicates the position of the substituent.

Table 2

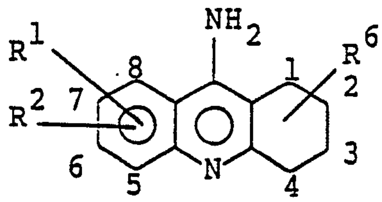

| $R^1$ | $R^2$ | $R^6$ |
|---|---|---|
| F(8) | H | H |
| F(7) | H | H |
| F(6) | H | H |
| F(5) | H | H |
| F(8) | H | $CH_3$(1) |
| F(8) | H | $CH_3$(2) |
| F(8) | H | $CH_3$(3) |
| F(8) | H | $CH_3$(4) |
| F(7) | H | $CH_3$(1) |
| F(7) | H | $CH_3$(2) |
| F(7) | H | $CH_3$(3) |
| F(7) | H | $CH_3$(4) |
| $CF_3$(6) | H | $CH_3$(1) |
| $CF_3$(7) | H | H |
| OH(8) | H | H |
| $NH_2$(7) | H | H |
| $CH_3CONH$ (7) | H | H |
| EtNH(7) | H | H |

- cont'd -

Table 2 (cont'd)

| $R^1$ | $R^2$ | $R^6$ |
|---|---|---|
| $CH_3S$(8) | H | H |
| $NO_2$(8) | H | H |
| F(6) | F(7) | H |
| F(6) | F(8) | H |
| F(6) | F(8) | $CH_3$(4) |
| F(6) | $CH_3$(7) | H |
| F(6) | F(7) | $CH_3$(2) |
| F(6) | F(8) | $CH_3$(2) |
| F(6) | $CH_3$(7) | $CH_3$(2) |
| $CF_3$(6) | H | H |
| F(6) | H | $CH_3$(1) |
| F(6) | H | $CH_3$(2) |
| F(6) | H | $CH_3$(3) |
| F(6) | H | $CH_3$(4) |

The numeral in parenthesis indicates the position of the substituent.

Table 3

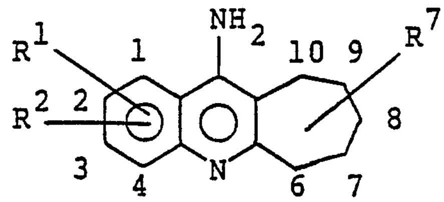

| $R^1$ | $R^2$ | $R^7$ |
|---|---|---|
| F(1) | H | H |
| F(2) | H | H |
| F(3) | H | H |
| F(4) | H | H |
| Cl(1) | H | $CH_3$(9) |
| Cl(1) | H | $CH_3$(8) |
| H | H | $CH_3$(9) |
| H | H | $CH_3$(8) |
| Cl(2) | H | $CH_3$(9) |
| Cl(2) | H | $CH_3$(8) |
| $CF_3$(2) | H | H |
| $CF_3$(2) | H | $CH_3$(9) |
| $CF_3$(2) | H | $CH_3$(8) |
| $OCH_3$(3) | H | $CH_3$(9) |
| $OCH_3$(3) | H | $CH_3$(8) |
| F(1) | H | $CH_3$(9) |
| F(1) | H | $CH_3$(8) |
| Cl(1) | H | $CH_3$(9) |
| Cl(1) | H | $CH_3$(8) |

- cont'd -

Table 3 (cont'd)

| $R^1$ | $R^2$ | $R^7$ |
|---|---|---|
| $OCH_3$(1) | H | $CH_3$(9) |
| $OCH_3$(1) | H | $CH_3$(8) |
| H | H | $CH_3$(6) |
| Cl(2) | H | $CH_3$(6) |
| $CF_3$(2) | H | $CH_3$(6) |
| $OCH_3$(3) | H | $CH_3$(6) |
| $OCH_3$(1) | H | $CH_3$(6) |
| F(1) | H | $CH_3$(6) |
| Cl(1) | H | $CH_3$(6) |
| $CH_3$(1) | H | $CH_3$(6) |
| $CF_3$(3) | H | H |
| F(1) | F(3) | H |
| F(2) | F(3) | H |
| F(3) | $CH_3$(2) | H |
| $CF_3$(3) | H | $CH_3$(6) |
| $CF_3$(3) | H | $CH_3$(9) |
| $CF_3$(3) | H | $CH_3$(8) |
| F(1) | F(3) | $CH_3$(9) |
| F(1) | F(3) | $CH_3$(8) |
| F(2) | F(3) | $CH_3$(9) |
| F(2) | F(3) | $CH_3$(8) |

The numeral in parenthesis indicates the position of the substituent.

Table 4

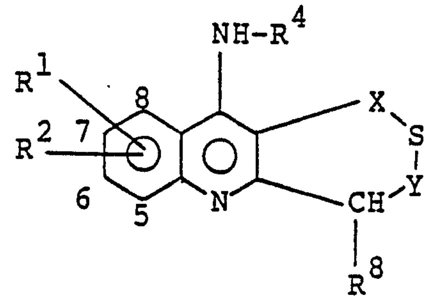

| $R^1$ | $R^2$ | $R^4$ | $R^8$ | X | Y |
|---|---|---|---|---|---|
| H | H | H | H | $CH_2$ | $CH_2$ |
| H | H | $CH_2CH_2Ph$ | H | $CH_2$ | $CH_2$ |
| F(8) | H | H | H | $CH_2$ | $CH_2$ |
| $OCH_3$(8) | H | H | H | $CH_2$ | $CH_2$ |
| $CH_3$(8) | H | H | H | $CH_2$ | $CH_2$ |
| F(6) | $CH_3$(7) | H | H | $CH_2$ | $CH_2$ |
| F(8) | H | H | H | $CH_2$ | $CHCH_3$ |
| F(8) | H | H | $CH_3$ | $CH_2$ | $CH_2$ |
| F(8) | H | H | H | $CHCH_3$ | $CH_2$ |
| H | H | H | H | $CH_2CH_2$ | --- |
| F(8) | H | H | H | $CH_2CH_2$ | --- |
| $CH_3$(8) | H | H | H | $CH_2CH_2$ | --- |
| $OCH_3$(8) | H | H | H | $CH_2CH_2$ | --- |
| Cl(8) | H | H | H | $CH_2CH_2$ | --- |
| F(6) | $CH_3$(7) | H | H | $CH_2CH_2$ | --- |
| F(8) | H | H | H | $CH_2CHCH_3$ | --- |
| F(6) | F(7) | H | H | $CH_2CH_2$ | --- |
| H | H | H | $CH_3$ | $CH_2CH_2$ | --- |

- cont'd -

Table 4 (cont'd)

| $R^1$ | $R^2$ | $R^4$ | $R^8$ | X | Y |
|---|---|---|---|---|---|
| F(8) | H | H | $CH_3$ | $CH_2CH_2$ | --- |
| H | H | $CH_2CH_2Ph$ | H | $CH_2CH_2$ | --- |
| F(6) | F(8) | H | H | $CH_2CH_2$ | --- |
| F(6) | $CF_3$(8) | H | H | $CH_2CH_2$ | --- |
| F(6) | H | H | $CH_3$ | $CH_2CH_2$ | --- |
| F(6) | H | H | H | $CH_2CH_2$ | --- |
| F(7) | H | H | H | $CH_2CH_2$ | --- |
| F(7) | H | H | $CH_3$ | $CH_2CH_2$ | --- |
| $CH_3$(8) | H | H | $CH_3$ | $CH_2CH_2$ | --- |
| $CH_3$(6) | H | H | H | $CH_2CH_2$ | --- |
| F(5) | H | H | H | $CH_2CH_2$ | --- |
| $CF_3$(6) | H | H | H | $CH_2CH_2$ | --- |
| H | H | H | H | --- | $CH_2CH_2$ |
| F(8) | H | H | H | --- | $CH_2CH_2$ |
| $CH_3$(8) | H | H | H | --- | $CH_2CH_2$ |
| $OCH_3$(8) | H | H | H | --- | $CH_2CH_2$ |
| F(6) | F(7) | H | H | --- | $CH_2CH_2$ |
| F(6) | F(8) | H | H | --- | $CH_2CH_2$ |
| F(6) | $CH_3$(7) | H | H | --- | $CH_2CH_2$ |
| H | H | H | H | --- | $CHCH_3CH_2$ |
| H | H | H | H | --- | $CH_2CHCH_3$ |
| H | H | H | $CH_3$ | --- | $CH_2CH_2$ |
| F(8) | H | H | $CH_3$ | --- | $CH_2CH_2$ |
| F(8) | H | H | H | --- | $CHCH_3CH_2$ |

- cont'd -

Table 4 (cont'd)

| $R^1$ | $R^2$ | $R^4$ | $R^8$ | X | Y |
|---|---|---|---|---|---|
| F(8) | H | H | H | --- | $CH_2CHCH_3$ |
| H | H | H | H | --- | $CH_2$ |
| F(8) | H | H | H | --- | $CH_2$ |
| $CH_3$(8) | H | H | H | --- | $CH_2$ |
| $OCH_3$(8) | H | H | H | --- | $CH_2$ |
| H | H | H | $CH_3$ | --- | $CH_2$ |
| F(8) | H | H | $CH_3$ | --- | $CH_2$ |
| F(6) | $CH_3$(7) | H | H | --- | $CH_2$ |
| H | H | H | H | $CH_2$ | --- |
| F(8) | H | H | H | $CH_2$ | --- |
| $CH_3$(8) | H | H | H | $CH_2$ | --- |
| $OCH_3$(8) | H | H | H | $CH_2$ | --- |
| H | H | $CH_2CH_2Ph$ | H | $CH_2$ | --- |
| F(8) | H | H | $CH_3$ | $CH_2$ | --- |
| F(6) | $CH_3$(7) | H | H | $CH_2$ | --- |
| H | H | H | H | --- | $CH_2CH_2CH_2$ |
| F(8) | H | H | H | --- | $CH_2CH_2CH_2$ |
| $CH_3$(8) | H | H | H | --- | $CH_2CH_2CH_2$ |
| H | H | $CH_2CH_2Ph$ | H | --- | $CH_2CH_2CH_2$ |
| H | H | H | H | $CH_2CH_2CH_2$ | --- |
| F(8) | H | H | H | $CH_2CH_2CH_2$ | --- |
| $CH_3$(8) | H | H | H | $CH_2CH_2CH_2$ | --- |
| F(6) | $CH_3$(7) | H | H | $CH_2CH_2CH_2$ | --- |

- cont'd -

Table 4 (cont'd)

| $R^1$ | $R^2$ | $R^4$ | $R^8$ | X | Y |
|---|---|---|---|---|---|
| H | H | H | H | $CH_2CH_2$ | $CH_2$ |
| H | H | H | H | $CH_2$ | $CH_2CH_2$ |
| F(8) | H | H | H | $CH_2CH_2$ | $CH_2$ |
| F(8) | H | H | H | $CH_2$ | $CH_2CH_2$ |

The numeral in parenthesis indicates the position of the substituent and "---" indicates a chemical bond.

Table 5

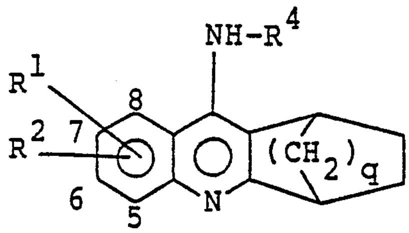

| $R^1$ | $R^2$ | $R^4$ | q |
|---|---|---|---|
| H | H | H | 1 |
| F(8) | H | H | 1 |
| F(7) | H | H | 1 |
| F(6) | H | H | 1 |
| F(5) | H | H | 1 |
| Cl(8) | H | H | 1 |
| Cl(6) | H | H | 1 |
| H | H | $CH_2CH_2Ph$ | 1 |
| F(6) | F(7) | H | 1 |
| F(6) | F(8) | H | 1 |
| F(6) | $CH_3$(7) | H | 1 |
| $CH_3$(8) | H | H | 1 |
| $CH_3$(6) | H | H | 1 |
| F(6) | F(7) | $CH_3$ | 1 |
| $OCH_3$(8) | H | H | 1 |
| $OCH_3$(6) | H | H | 1 |
| $CF_3$(7) | H | H | 1 |
| $CF_3$(6) | H | H | 1 |
| $NO_2$(7) | H | H | 1 |

- cont'd -

Table 5 (cont'd)

| $R^1$ | $R^2$ | $R^4$ | q |
|---|---|---|---|
| H | H | H | 2 |
| F(8) | H | H | 2 |
| F(7) | H | H | 2 |
| F(6) | H | H | 2 |
| F(5) | H | H | 2 |
| Cl(8) | H | H | 2 |
| Cl(6) | H | H | 2 |
| H | H | $CH_2CH_2Ph$ | 2 |
| F(6) | F(7) | H | 2 |
| F(6) | F(8) | H | 2 |
| F(6) | $CH_3$(7) | H | 2 |
| $CH_3$(8) | H | H | 2 |
| $CH_3$(6) | H | H | 2 |
| H | H | $CH_3$ | 2 |
| $OCH_3$(8) | H | H | 2 |
| $OCH_3$(6) | H | H | 2 |
| $CF_3$(7) | H | H | 2 |
| $CF_3$(6) | H | H | 2 |
| $NO_2$(7) | H | H | 2 |
| F(6) | F(7) | $CH_2CH_2Ph$ | 1 |
| H | H | $CH_3$ | 1 |
| $CH_3$(5) | $CH_3$(7) | H | 1 |

The numeral in parenthesis indicates the position of the substituent.

Table 6

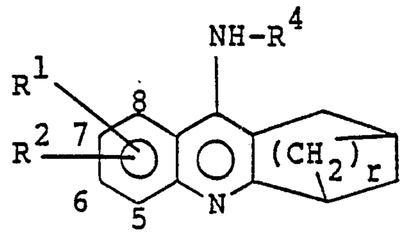

| $R^1$ | $R^2$ | $R^4$ | r |
|---|---|---|---|
| H | H | H | 1 |
| F(8) | H | H | 1 |
| F(7) | H | H | 1 |
| F(6) | H | H | 1 |
| F(5) | H | H | 1 |
| Cl(8) | H | H | 1 |
| Cl(6) | H | H | 1 |
| H | H | $CH_2CH_2Ph$ | 1 |
| F(6) | F(7) | H | 1 |
| F(6) | F(8) | H | 1 |
| F(6) | $CH_3$(7) | H | 1 |
| $CH_3$(8) | H | H | 1 |
| $CH_3$(6) | H | H | 1 |
| F(6) | F(7) | $CH_3$ | 1 |
| $OCH_3$(8) | H | H | 1 |
| $OCH_3$(6) | H | H | 1 |
| $CF_3$(7) | H | H | 1 |
| $CF_3$(6) | H | H | 1 |
| $NO_2$(7) | H | H | 1 |

- cont'd -

Table 6 (cont'd)

| $R^1$ | $R^2$ | $R^4$ | r |
|---|---|---|---|
| H | H | H | 2 |
| F(8) | H | H | 2 |
| F(7) | H | H | 2 |
| F(6) | H | H | 2 |
| F(5) | H | H | 2 |
| Cl(8) | H | H | 2 |
| Cl(6) | H | H | 2 |
| H | H | $CH_2CH_2Ph$ | 2 |
| F(6) | F(7) | H | 2 |
| F(6) | F(8) | H | 2 |
| F(6) | $CH_3$(7) | H | 2 |
| $CH_3$(8) | H | H | 2 |
| $CH_3$(6) | H | H | 2 |
| H | H | $CH_3$ | 2 |
| $OCH_3$(8) | H | H | 2 |
| $OCH_3$(6) | H | H | 2 |
| $CF_3$(7) | H | H | 2 |
| $CF_3$(6) | H | H | 2 |
| $NO_2$(7) | H | H | 2 |
| F(6) | F(7) | $CH_2CH_2Ph$ | 1 |
| H | H | $CH_3$ | 1 |
| F(8) | H | $CH_2CH_2Ph$ | 1 |
| F(6) | H | $CH_2CH_2Ph$ | 1 |

- cont'd -

Table 6 (cont'd)

| $R^1$ | $R^2$ | $R^4$ | r |
|---|---|---|---|
| F(8) | H | $CH_2CH_2Ph$ | 2 |
| F(6) | H | $CH_2CH_2Ph$ | 2 |

The numeral in parenthesis indicates the position of the substituent.

Table 7

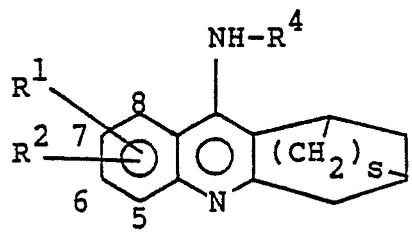

| $R^1$ | $R^2$ | $R^4$ | s |
|---|---|---|---|
| H | H | H | 1 |
| F(8) | H | H | 1 |
| F(7) | H | H | 1 |
| F(6) | H | H | 1 |
| F(5) | H | H | 1 |
| Cl(8) | H | H | 1 |
| Cl(6) | H | H | 1 |
| H | H | $CH_2CH_2Ph$ | 1 |

- cont'd -

Table 7 (cont'd)

| $R^1$ | $R^2$ | $R^4$ | s |
|---|---|---|---|
| F(6) | F(7) | H | 1 |
| F(6) | F(8) | H | 1 |
| F(6) | $CH_3$(7) | H | 1 |
| $CH_3$(8) | H | H | 1 |
| $CH_3$(6) | H | H | 1 |
| F(6) | F(7) | $CH_3$ | 1 |
| $OCH_3$(8) | H | H | 1 |
| $OCH_3$(6) | H | H | 1 |
| $CF_3$(7) | H | H | 1 |
| $CF_3$(6) | H | H | 1 |
| $NO_2$(7) | H | H | 1 |
| H | H | H | 2 |
| F(8) | H | H | 2 |
| F(7) | H | H | 2 |
| F(6) | H | H | 2 |
| F(5) | H | H | 2 |
| Cl(8) | H | H | 2 |
| Cl(6) | H | H | 2 |
| H | H | $CH_2CH_2Ph$ | 2 |
| F(6) | F(7) | H | 2 |
| F(6) | F(8) | H | 2 |
| F(6) | $CH_3$(7) | H | 2 |
| $CH_3$(8) | H | H | 2 |

- cont'd -

Table 7 (cont'd)

| $R^1$ | $R^2$ | $R^4$ | s |
|---|---|---|---|
| $CH_3$(6) | H | H | 2 |
| H | H | $CH_3$ | 2 |
| $OCH_3$(8) | H | H | 2 |
| $OCH_3$(6) | H | H | 2 |
| $CF_3$(7) | H | H | 2 |
| $CF_3$(6) | H | H | 2 |
| $NO_2$(7) | H | H | 2 |
| F(6) | F(7) | $CH_2CH_2Ph$ | 1 |
| H | H | $CH_3$ | 1 |
| F(8) | H | $CH_2CH_2Ph$ | 1 |
| F(6) | H | $CH_2CH_2Ph$ | 1 |
| F(8) | H | $CH_2CH_2Ph$ | 2 |
| F(6) | H | $CH_2CH_2Ph$ | 2 |

The numeral in parenthesis indicates the position of the substituent.

Table 8

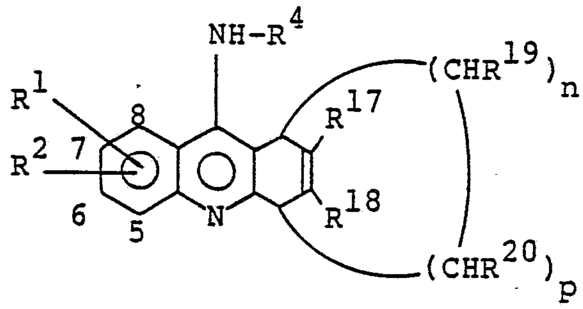

| $R^1$ | $R^2$ | $R^4$ | $R^{17}$ | $R^{18}$ | $R^{19}$ | $R^{20}$ | n | p |
|---|---|---|---|---|---|---|---|---|
| H | H | H | H | H | H | --- | 1 | 0 |
| F(8) | H | H | H | H | H | --- | 1 | 0 |
| F(7) | H | H | H | H | H | --- | 1 | 0 |
| F(6) | H | H | H | H | H | --- | 1 | 0 |
| F(5) | H | H | H | H | H | --- | 1 | 0 |
| Cl(8) | H | H | H | H | H | --- | 1 | 0 |
| Cl(7) | H | H | H | H | H | --- | 1 | 0 |
| Cl(6) | H | H | H | H | H | --- | 1 | 0 |
| $CH_3$(8) | H | H | H | H | H | --- | 1 | 0 |
| $CH_3$(6) | H | H | H | H | H | --- | 1 | 0 |
| $OCH_3$(8) | H | H | H | H | H | --- | 1 | 0 |
| $CH_3$(5) | $CH_3$(7) | H | H | H | H | --- | 1 | 0 |
| $CF_3$(6) | H | H | H | H | H | --- | 1 | 0 |
| F(6) | F(7) | H | H | H | H | --- | 1 | 0 |
| H | H | $CH_2CH_2Ph$ | H | H | H | --- | 1 | 0 |
| H | H | H | $CH_3$ | H | H | --- | 1 | 0 |
| H | H | H | H | $CH_3$ | H | --- | 1 | 0 |

- cont'd -

Table 8 (cont'd)

| $R^1$ | $R^2$ | $R^4$ | $R^{17}$ | $R^{18}$ | $R^{19}$ | $R^{20}$ | n | p |
|---|---|---|---|---|---|---|---|---|
| H | H | H | H | H | $CH_3$ | --- | 1 | 0 |
| $OCH_3$(6) | H | H | H | H | H | --- | 1 | 0 |
| H | H | H | H | H | H | H | 1 | 1 |
| F(8) | H | H | H | H | H | H | 1 | 1 |
| F(7) | H | H | H | H | H | H | 1 | 1 |
| F(6) | H | H | H | H | H | H | 1 | 1 |
| F(5) | H | H | H | H | H | H | 1 | 1 |
| Cl(8) | H | H | H | H | H | H | 1 | 1 |
| Cl(6) | H | H | H | H | H | H | 1 | 1 |
| H | H | $CH_2CH_2Ph$ | H | H | H | H | 1 | 1 |
| F(6) | F(7) | H | H | H | H | H | 1 | 1 |
| F(6) | F(8) | H | H | H | H | H | 1 | 1 |
| H | H | $CH_3$ | H | H | H | H | 1 | 1 |
| H | H | H | $CH_3$ | H | H | H | 1 | 1 |
| H | H | H | H | $CH_3$ | H | H | 1 | 1 |
| H | H | H | H | H | $CH_3$ | H | 1 | 1 |
| H | H | H | H | H | H | $CH_3$ | 1 | 1 |
| $CH_3$(8) | H | H | H | H | H | H | 1 | 1 |
| $CH_3$(6) | H | H | H | H | H | H | 1 | 1 |
| Cl(7) | H | H | H | H | H | H | 1 | 1 |
| $OCH_3$(8) | H | H | H | H | H | H | 1 | 1 |
| $OCH_3$(6) | H | H | H | H | H | H | 1 | 1 |
| $CF_3$(6) | H | H | H | H | H | H | 1 | 1 |

- cont'd -

Table 8 (cont'd)

| $R^1$ | $R^2$ | $R^4$ | $R^{17}$ | $R^{18}$ | $R^{19}$ | $R^{20}$ | n | p |
|---|---|---|---|---|---|---|---|---|
| F(6) | $CH_3$(7) | H | H | H | H | H | 1 | 1 |
| F(6) | H | $CH_3$ | H | H | H | H | 1 | 1 |
| F(6) | H | H | $CH_3$ | H | H | H | 1 | 1 |
| F(6) | H | H | H | $CH_3$ | H | H | 1 | 1 |
| F(6) | H | H | H | H | $CH_3$ | H | 1 | 1 |
| F(6) | H | H | H | H | H | $CH_3$ | 1 | 1 |
| Cl(6) | H | $CH_3$ | H | H | H | H | 1 | 1 |
| Cl(6) | H | H | $CH_3$ | H | H | H | 1 | 1 |
| Cl(6) | H | H | H | $CH_3$ | H | H | 1 | 1 |
| Cl(6) | H | H | H | H | $CH_3$ | H | 1 | 1 |
| Cl(6) | H | H | H | H | H | $CH_3$ | 1 | 1 |
| H | H | H | H | H | H | H | 2 | 1 |
| F(8) | H | H | H | H | H | H | 2 | 1 |
| F(7) | H | H | H | H | H | H | 2 | 1 |
| F(6) | H | H | H | H | H | H | 2 | 1 |
| F(5) | H | H | H | H | H | H | 2 | 1 |
| Cl(8) | H | H | H | H | H | H | 2 | 1 |
| Cl(6) | H | H | H | H | H | H | 2 | 1 |
| F(6) | F(7) | H | H | H | H | H | 2 | 1 |
| F(6) | F(8) | H | H | H | H | H | 2 | 1 |
| F(6) | $CH_3$(7) | H | H | H | H | H | 2 | 1 |
| $CH_3$(8) | H | H | H | H | H | H | 2 | 1 |

- cont'd -

Table 8 (cont'd)

| $R^1$ | $R^2$ | $R^4$ | $R^{17}$ | $R^{18}$ | $R^{19}$ | $R^{20}$ | n | p |
|---|---|---|---|---|---|---|---|---|
| $CH_3$(6) | H | H | H | H | H | H | 2 | 1 |
| $OCH_3$(8) | H | H | H | H | H | H | 2 | 1 |
| $CH_3$(5) | $CH_3$(7) | H | H | H | H | H | 1 | 1 |
| $CF_3$(6) | H | H | H | H | H | H | 2 | 1 |

The numeral is parenthesis indicates the position of the substituent.

Table 9

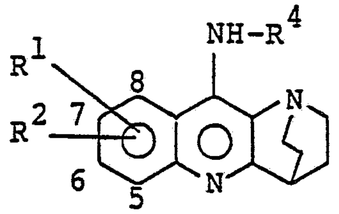

| $R^1$ | $R^2$ | $R^4$ |
|---|---|---|
| H | H | H |
| F(8) | H | H |
| F(7) | H | H |
| F(6) | H | H |
| Cl(8) | H | H |
| Cl(7) | H | H |
| Cl(6) | H | H |
| F(5) | H | H |
| $CF_3$(7) | H | H |
| $CF_3$(6) | H | H |
| $CH_3$(8) | H | H |
| $CH_3$(6) | H | H |
| $OCH_3$(8) | H | H |
| $OCH_3$(6) | H | H |
| H | H | $CH_2CH_2Ph$ |
| F(8) | H | $CH_2CH_2Ph$ |
| F(6) | F(7) | H |
| F(6) | F(8) | H |

- cont'd -

Table 9 (cont'd)

| $R^1$ | $R^2$ | $R^4$ |
|---|---|---|
| F(6) | $CH_3$(7) | H |
| F(8) | H | $CH_3$ |

The numeral is parenthesis indicates the position of the substituent.

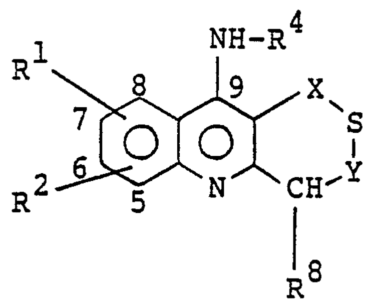

9-amino-2-thia-1,2,3,4-tetrahydroacridine,
9-amino-8-fluoro-2-thia-1,2,3,4-tetrahydroacridine,
9-amino-8-methyl-2-thia-1,2,3,4-tetrahydroacridine,
9-amino-8-methoxy2-thia-1,2,3,4-tetrahydroacridine,
9-amino-6-fluoro-7-methyl-2-thia-1,2,3,4-tetrahydroacridine,
9-amino-1-thia-1,2,3,4-tetrahydroacridine,
9-amino-3-thia-1,2,3,4-tetrahydroacridine,
9-amino-8-fluoro-1-thia-1,2,3,4-tetrahydroacridine,
9-amino-8-fluoro-3-thia-1,2,3,4-tetrahydroacridine,
9-amino-8-methoxy-1-thia-1,2,3,4-tetrahydroacridine,
9-amino-8-methyl-1-thia-1,2,3,4-tetrahydroacridine,
9-amino-8-methyl-3-thia-1,2,3,4-tetrahydroacridine,
9-amino-6-fluoro-7-methyl-1-thia-1,2,3,4-tetrahydroacridine,
9-amino-6-fluoro-7-methyl-3-thia-1,2,3,4-tetrahydroacridine,
9-amino-1-thia-2,3-dihydro-1H-cyclopenta[b]quinoline,
9-amino-2-thia-2,3-dihydro-1H-cyclopenta[b]quinoline,
9-amino-8-fluoro-1-thia-2,3-dihydro-1H-cyclopenta[b]quinoline,
9-amino-8-fluoro-2-thia-2,3-dihydro-1H-cyclopenta[b]quinoline,
9-amino-4,8-dimethyl-3-thia-1,2,3,4-tetrahydroacridine,
9-amino-8-fluoro-4-methyl-3-thia-1,2,3,4-tetrahydroacridine,
9-amino-7-fluoro-4-methyl-3-thia-1,2,3,4-tetrahydroacridine,
9-amino-4-methyl-3-thia-1,2,3,4-tetrahydroacridine,
9-amino-2-methyl-1-thia-1,2,3,4-tetrahydroacridine,
9-amino-2,8-dimethyl-1-thia-1,2,3,4-tetrahydroacridine,
9-amino-8-methyl-1-thia-2,3-dihydro-1H-cyclopenta[b]quinoline,
9-amino-6-fluoro-1-thia-1,2,3,4-tetrahydroacridine,

9-amino-6-fluoro-3-thia-1,2,3,4-tetrahydroacridine,
9-amino-7-fluoro-1-thia-1,2,3,4-tetrahydroacridine,
9-amino-7-fluoro-3-thia-1,2,3,4-tetrahydroacridine,
9-amino-6-trifluoromethyl-1-thia-1,2,3,4-tetrahydroacridine,
9-amino-6-trifluoromethyl-3-thia-1,2,3,4-tetrahydroacridine,
9-amino-6,7-difluoro-2-thia-1,2,3,4-tetrahydroacridine,
9-amino-6,7-difluoro-1-thia-1,2,3,4-tetrahydroacridine,
9-amino-6,7-difluoro-3-thia-1,2,3,4-tetrahydroacridine,
9-amino-5-fluoro-1-thia-1,2,3,4-tetrahydroacridine,
9-amino-5-fluoro-3-thia-1,2,3,4-tetrahydroacridine,
9-amino-7-chhloro-1-thia-1,2,3,4-tetrahydroacridine,
9-amino-7-chloro-3-thia-1,2,3,4-tetrahydroacridine,
9-amino-8-chloro-3-thia-1,2,3,4-tetrahydroacridine,
9-amino-7-methyl-1-thia-1,2,3,4-tetrahydroacridine,
9-amino-7-methyl-3-thia-1,2,3,4-tetrahydroacridine,
9-amino-8-methyl-1-thia-1,2,3,4-tetrahydroacridine,
9-amino-8-methyl-3-thia-1,2,3,4-tetrahydroacridine,
9-amino-6,7-difluoro-1-thia-1,2,3,4-tetrahydroacridine,
9-amino--6,7-difluoro-3-thia-1,2,3,4-tetrahydroacridine.

Practical and presently preferred embodiments of the invention are illustratively shown in the following Examples, which are not intended to limit the scope of the invention thereto.

Example 1

Preparation of 9-amino-8-fluoro-1,2,3,4-tetrahydroacridine

Under a nitrogen atmosphere anhydrous aluminum chloride (2.16 g, 16.2 mmol) was added to 1,2-dichloroethane (60 ml) at room temperature with stirring. 2-Amino-6-fluorobenzonitrile (2.00 g, 14.7 mmol) was added to the reaction mixture. A 1,2-dichloroethane (10 ml) solution of cyclohexanone (1.44 g, 14.7 mmol) was added dropwise to the reaction mixture under ice-cooling and then the reaction mixture was heated under reflux for one hour. A mixture of tetrahydrofuran (60 ml) and water (30 ml) was added dropwise to the reaction mixture under ice-cooling. An aqueous sodium hydroxide solution was added dropwise to the mixture under ice-cooling until the aqueous solution was made basic to litmus, followed by stirring for 0.5 hour at room temperature. The mixture was extracted three times with ethyl acetate. The extract was washed with an aqueous sodium chloride solution, dried over anhydrous magnesium sulfate. The solvent was concentrated, followed by stirring for one hour under ice-cooling. A crystalline precipitate was filtered with suction and then washed successively with cold toluene and a cold mixture of ether and n-hexane, followed by drying to give 9-amino-8-fluoro-1,2,3,4-tetrahydroacridine, melting point 174 - 175°C.

Example 2

Preparation of 9-amino-6-fluoro-1,2,3,4-tetrahydroacridine

Under a nitrogen atmosphere a mixture of 2-amino-4-fluorobenzonitrile (2.00 g, 14.7 mmol), cyclohexanone (4.00 g, 40.8 mmol) and anhydrous zinc chloride (2.00 g, 14.7 mmol) was heated for 15 minutes at 140 - 150°C with stirring. N,N-dimethylformamide (20 ml) was added to the reaction mixture, followed by stirring until the solid mass was dissolved. An aqueous sodium hydroxide solution was added dropwise to the reaction mixture under ice-cooling. The resulting mixture was made alkaline and poured into water and extracted three times with ethyl acetate. The extract was washed twice with an aqueous sodium chloride solution, dried over anhydrous magnesium sulfate and concentrated. The residue was purified by column chromatography on silica gel to give 9-amino-6-fluoro-1,2,3,4-tetrahydroacridine, melting point 222 - 223°C.

Example 3

Preparation of 9-amino-1,4-dihydro-1,4-ethanoacridine

Under a nitrogen atmosphere a mixture of 2-aminobenzonitrile (1.00 g 8.5 mmol), bicyclo[2.2.2]oct-5-en-2-one (1.50 g, 12.3 mmol) and anhydrous zinc chloride (2.00 g, 14.7 mmol) was heated for one hour at 160 - 170°C with stirring. N,N-dimethylformamide (10 ml) was added to the reaction mixture, followed by stirring until the solid mass was dissolved. An aqueous sodium hydroxide solution was added dropwise to the reaction mixture under ice-cooling. The resulting mixture was made alkaline and poured into water and extracted three times with ethyl acetate. The extract was washed twice with an aqueous sodium chloride solution, dried over anhydrous magnesium sulfate and concentrated. The residue was purified by column chromatography on silica gel to give 9-amino-1,4-dihydro-1,4-ethanoacridine, melting point 218 - 219°C.

Example 4

Preparation of 9-amino-1,4-dihydro-1,4-ethanoacridine

Under a nitrogen atmosphere anhydrous aluminum chloride (2.48 g, 18.6 mmol) was added to 1,2-dibromoethane (40 ml) at room temperature with stirring. A 1,2-dibromoethane (10 ml) solution of 2-aminobenzonitrile (2.00 g, 16.9 mmol) was added dropwise to the reaction mixture at 70 - 80°C. A 1,2-dibromoethane (10 ml) solution of bicyclo[2.2.2]oct-5-en-2-one (2.27 g, 18.6 mmol) was added dropwise to the reaction mixture at 120 - 130°C and then the reaction mixture was heated under reflux for one hour. A mixture of tetrahydrofuran (40 ml) and water (30 ml) was added dropwise to the reaction mixture under ice-cooling. An aqueous sodium hydroxide solution was added dropwise to the mixture under ice-cooling. The resulting mixture was made alkaline and stirred for 0.5 hour at room temperature. The mixture was extracted three times with ethyl acetate. The extract was washed with a saturated aqueous sodium chloride solution, dried over anhydrous magnesium sulfate. Evaporation of solution gave crystalline product, which was purified by column chromatography on silica gel to give 9-amino-1,4-dihydro-1,4-ethanoacridine, melting point 218 - 219°C.

Example 5

Preparation of 9-amino-1,2,3,4-tetrahydro-1,4-methanoacridine hydrochloride

A isopropyl alcohol solution of hydrogen chloride was added dropwised to a isopropyl alcohol (30 ml) solution of 9-amino-1,2,3,4-tetrahydro-1,4-methanoacridine (1.00 g) (Example 54) until the solution was made acid. Evaporation of solvent gave crystalline product, which was filtered with suction and then washed with ether, followed by drying to give 9-amino-1,2,3,4-tetrahydro-1,4-methanoacridine hydrochloride, melting point 183 - 190°C.

Examples 6 - 173

In the same manner as in Example 1, 2, 3, or 4, there were produced the compounds as shown in Table 10 - 22.

Also hydrochloride of Examples 1, 2, 3, or 6 - 78, 87 - 89, 91, 93 - 155, or 158 - 173 were synthesized in the same manner as in Example 5.

Table 10

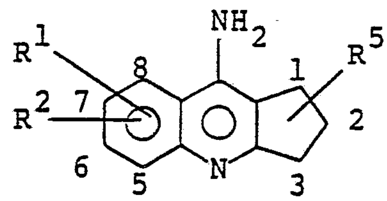

| Example No. | $R^1$ | $R^2$ | $R^5$ | m.p. °C |
|---|---|---|---|---|
| 6 | F(8) | H | H | 177 - 180 |
| 7 | F(7) | H | H | 235 - 236 |
| 8 | F(6) | H | H | 230 (decomp.) |
| 9 | F(6) | F(7) | H | 256 (decomp.) |
| 10 | F(6) | F(8) | H | 154 - 156 |
| 11 | F(8) | H | $CH_3$(3) | 153 - 155 |
| 12 | F(5) | H | H | 259 - 260 |
| 13 | F(8) | H | $C_2H_5$(3) | 174 - 176 |
| 14 | $CH_3$(8) | H | $CH_3$(2) | 147 - 149 |
| 15 | $CH_3$(8) | H | $CH_3$(3) | 97 - 102 |
| 16 | F(6) | $CH_3$(7) | $CH_3$(3) | 198 - 202 |
| 17 | $CF_3$(7) | H | H | 220 - 223 |
| 18 | Cl(7) | H | $CH_3$(3) | 210 |
| 19 | H | H | $CH_3$(2) | 175 - 177 |
| 20 | H | H | $CH_3$(3) | 161 - 163 |
| 21 | H | H | $C_2H_5$(3) | 131 - 135 |

The numeral in parenthesis indicates the position of the substituent.

Table 11

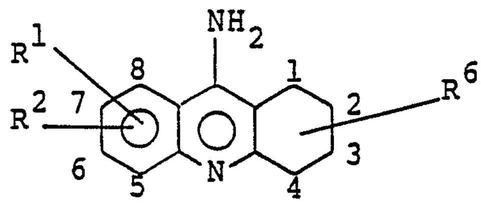

| Example No. | $R^1$ | $R^2$ | $R^6$ | m.p. °C |
|---|---|---|---|---|
| 22 | F(7) | H | H | 233 - 235 |
| 23 | F(5) | H | H | 242 - 245 |
| 24 | F(8) | H | $CH_3$(1) | 150 - 151 |
| 25 | F(8) | H | $CH_3$(2) | 218 - 221 |
| 26 | F(8) | H | $CH_3$(3) | 233 - 235 |
| 27 | F(8) | H | $CH_3$(4) | 144 - 146 |
| 28 | F(7) | H | $CH_3$(1) | 235 - 242 |
| 29 | F(7) | H | $CH_3$(2) | 226 - 230 |
| 30 | F(7) | H | $CH_3$(3) | 235 - 237 |
| 31 | F(7) | H | $CH_3$(4) | 197 - 202 |
| 32 | F(6) | F(7) | H | 252 - 256 |
| 33 | F(6) | F(8) | H | 182 - 184 |
| 34 | F(6) | $CH_3$(7) | H | 255 - 261 |
| 35 | F(6) | $CH_3$(7) | $CH_3$(2) | 243 - 244 |
| 36 | $CF_3$(6) | H | H | 250 - 255 |
| 157 | F(8) | H | H | 265 - 269* |

The numeral in parenthesis indicates the position of the substituent.

*: Hydrochloride.

Table 12

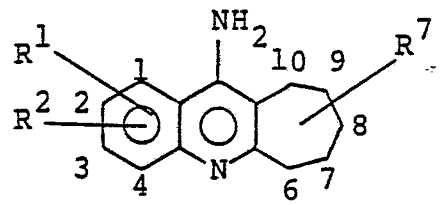

| Example No. | $R^1$ | $R^2$ | $R^7$ | m.p. °C |
|---|---|---|---|---|
| 37 | F(1) | H | H | 201 - 202 |
| 38 | F(2) | F(3) | H | 270 - 273 |
| 39 | F(3) | H | H | 209 - 212 |
| 40 | F(1) | F(3) | H | 125 - 126 |
| 41 | Cl(1) | H | $CH_3$(6) | 130 - 132 |
| 42 | Cl(1) | H | $CH_3$(7) | 144 - 148 |
| 43 | H | H | $CH_3$(6) | 135 |
| 44 | H | H | $CH_3$(7) | 187 - 191 |
| 45 | Cl(2) | H | $CH_3$(7) | 249 - 251 |
| 46 | Cl(2) | H | $CH_3$(6) | 134 - 135 |
| 47 | $CF_3$(2) | H | $CH_3$(6) | 128 - 129 |
| 48 | $CF_3$(2) | H | $CH_3$(7) | 205 - 210 |
| 49 | $OCH_3$(3) | H | $CH_3$(6) | 173 - 175 |
| 50 | $OCH_3$(3) | H | $CH_3$(7) | 185 - 187 |
| 51 | F(1) | H | $CH_3$(6) | 174 - 175 |
| 52 | $CH_3$(1) | H | $CH_3$(6) | 115 - 117 |
| 53 | F(1) | H | $CH_3$(7) | 186 - 190 |
| 156 | F(4) | H | H | 285 - 289* |

*: Hydrochloride.

The numeral in parenthesis indicates the position of the substituent.

Table 13

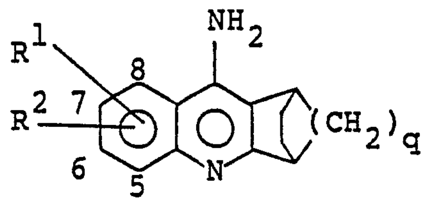

| Example No. | $R^1$ | $R^2$ | q | m.p. °C |
|---|---|---|---|---|
| 54 | H | H | 1 | 180 - 181 |
| 55 | F(8) | H | 1 | 168 - 169 |
| 56 | F(7) | H | 1 | 244 - 247 |
| 57 | F(5) | H | 1 | 227 - 228 |
| 58 | Cl(8) | H | 1 | 143 - 145 |
| 59 | Cl(7) | H | 1 | 176 - 178 |
| 60 | F(6) | F(7) | 1 | 208 - 209 |
| 61 | F(6) | $CH_3$(7) | 1 | 193 - 195 |
| 62 | $CH_3$(8) | H | 1 | 137 - 140 |
| 63 | $CH_3$(6) | H | 1 | 106 - 109 |
| 64 | $OCH_3$(8) | H | 1 | 113 - 116 |
| 65 | $OCH_3$(6) | H | 1 | 97 - 98 |
| 66 | $CF_3$(7) | H | 1 | 200 - 201 |
| 67 | $CF_3$(6) | H | 1 | 171 - 173 |
| 68 | $NO_2$(7) | H | 1 | 218 - 220 |
| 69 | H | H | 2 | 215 - 222 |
| 70 | F(8) | H | 2 | 247 - 250 |

- cont'd -

Table 13 (cont'd)

| Example No. | $R^1$ | $R^2$ | q | m.p. °C |
|---|---|---|---|---|
| 71 | F(7) | H | 2 | 233 - 235 |
| 72 | $CF_3$(6) | H | 2 | 170 - 175 |
| 73 | $CH_3$(5) | $CH_3$(7) | 1 | 170 - 176 |
| 88 | F(6) | H | 1 | 284 - 287 |

The numeral in parenthesis indicates the position of the substituent.

Table 14

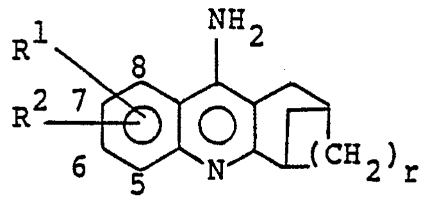

| Example No. | $R^1$ | $R^2$ | r | m.p. °C |
|---|---|---|---|---|
| 74 | H | H | 2 | 165 - 167 |
| 75 | F(7) | H | 2 | 184 - 185 |
| 76 | F(8) | H | 2 | 170 - 175 |
| 77 | $CH_3$(8) | H | 2 | 198 - 203 |
| 79 | F(6) | H | 2 | 155 - 160* (decomp.) |
| 80 | H | H | 1 | 294 - 298* (decomp.) |
| 81 | F(8) | H | 1 | 257 - 260* (decomp.) |
| 169 | F(6) | H | 1 | 204 - 208 |
| 170 | $CH_3$(8) | H | 1 | 126 - 129 |
| 171 | Cl(8) | H | 1 | 170 - 172 |
| 172 | F(6) | F(7) | 1 | 197 - 200 |
| 173 | Cl(6) | H | 1 | 181 - 184 |

*: Hydrochloride

The numeral in parenthesis indicates the position of the substituent.

Table 15

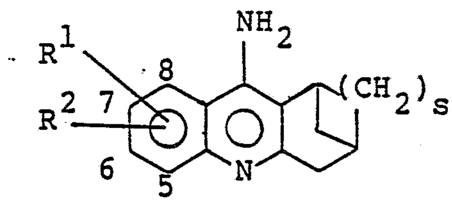

| Example No. | $R^1$ | $R^2$ | s | m.p. °C |
|---|---|---|---|---|
| 82 | H | H | 2 | 267 - 275* |
| 83 | F(8) | H | 2 | 267 - 270* |
| 84 | F(6) | H | 2 | 273 - 277* (decomp.) |
| 85 | F(6) | F(8) | 2 | 280 - 284* (decomp.) |
| 86 | $CH_3$(8) | H | 2 | 250 - 252* (decomp.) |

The numeral in parenthesis indicates the position of the substituent.

*: Hydrochloride.

Table 16

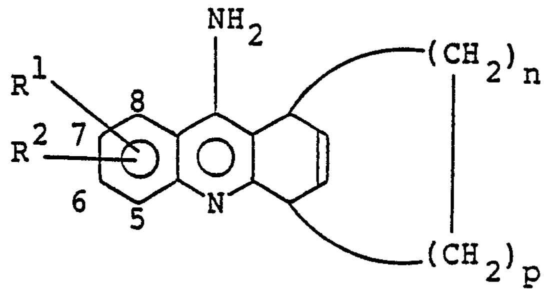

| Example No. | $R^1$ | $R^2$ | n | p | m.p. °C |
|---|---|---|---|---|---|
| 87 | F(8) | H | 1 | 1 | 199 - 205 |
| 89 | F(7) | H | 1 | 1 | 184 - 188 |
| 90 | F(6) | H | 1 | 1 | 202 - 207* (decomp.) |
| 92 | Cl(8) | H | 1 | 1 | 220 - 223* (decomp.) |
| 93 | Cl(7) | H | 1 | 1 | 239 - 241 (decomp.) |
| 95 | $CH_3$(8) | H | 1 | 1 | 149 - 150 |
| 96 | $CH_3$(5) | $CH_3$(7) | 1 | 1 | 120 - 125 |
| 97 | $OCH_3$(8) | H | 1 | 1 | 150 - 155 |
| 98 | F(6) | $CH_3$(7) | 1 | 1 | 220 - 221 |
| 99 | H | H | 1 | 0 | 177 - 182 |
| 100 | F(8) | H | 1 | 0 | 182 - 184 |
| 101 | $CH_3$(8) | H | 1 | 0 | 136 - 142 |
| 102 | $CH_3$(5) | $CH_3$(7) | 1 | 0 | 218 - 219 |
| 91 | F(6) | F(7) | 1 | 1 | 222 - 224 (decomp.) |

- cont'd -

Table 16 (cont'd)

| Example No. | $R^1$ | $R^2$ | n | p | m.p. °C |
|---|---|---|---|---|---|
| 94 | Cl(6) | H | 1 | 1 | 231 - 234 (decomp.) |

*: Hydrochloride.

The numeral is parenthesis indicates the position of the substituent.

Table 17

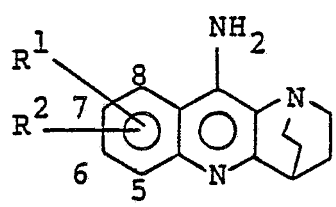

| Example No. | $R^1$ | $R^2$ | m.p. °C |
|---|---|---|---|
| 103 | H | H | 256 - 258 |
| 104 | F(8) | H | 139 - 141 |
| 105 | $CH_3$(8) | H | 172 - 175 |
| 106 | $CH_3$(6) | H | 230 - 231 |
| 107 | $OCH_3$(8) | H | 221 - 222 |
| 108 | F(6) | $CH_3$(7) | 264 - 265 |
| 109 | F(7) | H | 225 - 227 |

The numeral in parenthesis indicates the position of the substituent.

Table 18

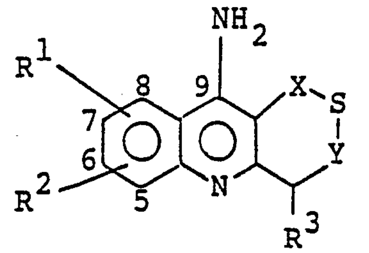

| Example No. | Compound | NMR ($CDCl_3$, TMS) δ: |
|---|---|---|
| 110 | 9-amino-2-thia-1,2,3,4-tetrahydroacridine | 2.96-3.02(2H, m), 3.30-3.35 (2H, m), 3.65(2H, s), 5.36 (2H, brs, $NH_2$), 6.96-7.04 (1H, m), 7.42-7.51(1H, m), 7.65(1H, dd, J=8.6Hz, J=1.0Hz) |
| 111 | 9-amino-8-fluoro-2-thia-1,2,3,4-tetrahydroacridine | 2.96-3.02(2H, m), 3.30-3.35 (2H, m), 3.65(2H, s), 5.36 (2H, brs, $NH_2$), 6.96-7.04 (1H, m), 7.42-7.51(1H, m), 7.65(1H, dd, J=8.6Hz, J=1.0Hz) |
| 112 | 9-amino-8-methyl-2-thia-1,2,3,4-tetrahydroacridine | 2.92-3.00(5H, m), 3.31(2H, t, J=6.0Hz), 3.63(2H, s), 5.02 (2H, brs, $NH_2$), 7.10(1H, d, J=6.9Hz), 7.38-7.45(1H, m), 7.70-7.74(1H, m) |

- cont'd -

Table 18 (cont'd)

| Example No. | Compound | NMR ($CDCl_3$, TMS) δ: |
| --- | --- | --- |
| 113 | 9-amino-8-methoxy-2-thia-1,2,3,4-tetrahydroacridine | 2.88-3.01(2H, m), 3.28-3.32 (2H, m), 3.62(2H, m), 4.00 (3H, s), 5.99(2H, brs, $NH_2$), 6.70(1H, dd, J=5.9Hz, J=3.0Hz), 7.40-7.47(2H, m) |
| 114 | 9-amino-6-fluoro-7-methyl-2-thia-1,2,3,4-tetrahydro-acridine | 2.45(3H, s), 2.98-3.03 (2H, m), 3.30-3.34(2H, m), 3.70 (2H, s), 4.64(2H, brs, $NH_2$), 7.47-7.50(2H, m) |
| 115 | 9-amino-1-thia-1,2,3,4-tetrahydro-acridine | 2.24-2.32(2H, m), 3.11(4H, t, J=6.3Hz), 4.88(2H, brs, $NH_2$), 7.37-7.43(1H, m), 7.53-7.60(1H, m), 7.64-7.67 (1H, m), 7.87-7.91(1H, m) |
| 116 | 9-amino-3-thia-1,2,3,4-tetrahydroacridine | 2.93(2H, m), 3.05(2H, m), 4.01(2H, s), 4.74(2H, brs, $NH_2$), 7.39-7.45(1H, m), 7.58-7.64(1H, m), 7.71(1H, |

- cont'd -

Table 18 (cont'd)

| Example No. | Compound | NMR ($CDCl_3$, TMS) δ: |
|---|---|---|
| | | d, J=8.3Hz), 7.89(1H, d, J=7.6Hz) |
| 117 | 9-amino-8-fluoro-1-thia-1,2,3,4-tetrahydroacridine | 2.22-2.31(2H, m), 3.05-3.13 (4H, m), 5.52(2H, brs, $NH_2$), 6.95-7.03(1H, m), 7.38-7.46 (1H, m), 7.64(1H, dd, J=8.6Hz, J=1.0Hz) |
| 118 | 9-amino-8-fluoro-3-thia-1,2,3,4-tetrahydroacridine | 2.83-2.87(2H, m), 3.03-3.08 (2H, m), 3.97(2H, s), 5.35 (2H, brs, $NH_2$), 6.96-7.04 (1H, m), 7.42-7.50(1H, m), 7.64(1H, dd, J=8.6Hz, J=1.0Hz) |
| 119 | 9-amino-8-methoxyl-thia-1,2,3,4-tetra-acridine | 2.20-2.29(2H, m), 3.02-3.10 (4H, m), 3.98(3H, s), 6.13 (2H, brs, $NH_2$), 6.69(1H, dd, J=7.3Hz, J=1.7Hz), 7.36-7.46 (2H, m) |

- cont'd -

Table 18 (cont'd)

| Example No. | Compound | NMR ($CDCl_3$, TMS) δ: |
|---|---|---|
| 120 | 9-amino-8-methyl-1-thia-1,2,3,4-tetra-acridine | 2.23-2.30(2H, m), 2.96(3H, s), 3.03-3.11(4H, m), 5.23 (2H, brs, $NH_2$), 7.08-7.11 (1H, m), 7.34-7.40(1H, m), 7.69-7.72(1H, m) |
| 121 | 9-amino-8-methyl-3-thia-1,2,3,4-tetra-hydroacridine | 2.84(2H, t, J=5.9Hz), 2.97 (3H, s), 3.05(2H, t, J=5.9Hz), 3.98(2H, s), 5.01 (2H, brs, $NH_2$), 7.11 (1H, d, J=6.6Hz), 7.41(1H, dd, J=8.3Hz, J=6.6Hz), 7.71(1H, d, J=8.3Hz) |
| 122 | 9-amino-6-fluoro-7-methyl-1-thia-1,2,3,4-tetrahydro-acridine | 2.21-2.31(2H, m), 2.44(3H, s), 3.05-3.12(4H, m), 4.82 (2H, brs, $NH_2$), 7.42-7.49 (2H, m) |
| 123 | 9-amino-6-fluoro-7-methyl- | 2.45(3H, s), 2.89-2.96(2H, m), 3.02-3.06(2H, m), |

- cont'd -

Table 18 (cont'd)

| Example No. | Compound | NMR ($CDCl_3$, TMS) δ: |
|---|---|---|
| | 3-thia-1,2,3,4-tetrahydro-acridine | 3.98(2H, s), 4.64 (2H, brs, $NH_2$), 7.44-7.50 (2H, m) |
| 124 | 9-amino-1-thia-2,3-dihydro-1H-cyclopenta[b]-quinoline | 3.42-3.55(4H, m), 4.50(2H, brs, $NH_2$), 7.39-7.47(2H, m), 7.53-7.61(1H, m), 7.61-7.69 (1H, m), 7.88-7.95(1H, m) |
| 125 | 9-amino-2-thia-2,3-dihydro-1H-cyclopenta[b]-quinoline | 4.16(2H, s), 4.41(2H, s), 4.68(2H, brs, $NH_2$), 7.42-7.48(1H, m), 7.61-7.67 (1H, m), 7.72-7.76(1H, m), 7.92-7.95(1H, m) |
| 126 | 9-amino-8-fluoro-1-thia-2,3-dihydro-1H-cyclopenta-[b]quinoline | 3.48(4H, s), 5.10(2H, brs, $NH_2$), 6.96-7.05(1H, m), 7.37-7.45(1H, m), 7.66(1H, d, J=8.6Hz) |
| 127 | 9-amino-8-fluoro-2-thia- | 4.10(2H, s), 4.38(2H, s), 5.28(2H, brs, $NH_2$), 7.45- |

- cont'd -

Table 18 (cont'd)

| Example No. | Compound | NMR ($CDCl_3$, TMS) δ: |
|---|---|---|
| | 2,3-dihydro-1H-cyclopenta-[b]quinoline | 7.54(1H, m), 7.68(1H, d, J=8.6Hz) |
| 128 | 9-amino-4,8-dimethyl-3-thia-1,2,3,4-tetrahydroacridine | 1.78(3H, d, J=7.3Hz), 2.75-3.22(7H, m), 4.13(1H, q, J=7.1Hz), 4.97(2H, brs, $NH_2$), 7.09-7.74(3H, m) |
| 129 | 9-amino-8-fluoro-4-methyl-3-thia-1,2,3,4-tetrahydro-acridine | 1.78(3H, d, J=7.3Hz), 2.79-3.21(4H, m), 4.12(1H, q, J=7.3Hz), 5.33(2H, brs, $NH_2$), 6.95-7.68(3H, m) |
| 130 | 9-amino-7-fluoro-4-methyl-3-thia-1,2,3,4-tetrahydro-acridine | 1,79(3H, d, J=6.9Hz), 2.87-3.19(4H, m), 4.15(1H, q, J=7.0Hz), 4.54(2H, brs, $NH_2$), 7.27-7.41(2H, m), 7.88-7.93 (1H, m) |
| 131 | 9-amino-4-methyl-3-thia- | 1.34(3H, d, J=6.9Hz), 1.92-2.05(2H, m), 2.50-2.66(2H, |

- cont'd -

Table 18 (cont'd)

| Example No. | Compound | NMR ($CDCl_3$, TMS) δ: |
|---|---|---|
| | 1,2,3,4-tetra-acridine | m), 3.01(1H, q, J=6.9Hz), 4.19(1H, brs), 6.48(1H, brs), 6.60-7.89(4H, m) |
| 132 | 9-amino-2-methyl-1-thia-1,2,3,4-tetra-acridine | 1.47(3H, d, J=6.6Hz), 1.86-2.00(1H, m), 2.31-2.41(1H, m), 3.02-3.24(2H, m), 3.48-3.56(1H, m), 4.58(2H, brs, $NH_2$), 7.37-7.43(1H, m), 7.53-7.67(2H, m), 7.87-7.90 (1H, m) |
| 133 | 9-amino-2,8-dimethyl-1-thia-1,2,3,4-tetra-acridine | 1,46(3H, d, J=6.9Hz), 1.84-1.97(1H, m), 2.29-2.40(1H, m), 2.96-3.20(5H, m), 3.45-3.51(1H, m), 5.21(2H, brs, $NH_2$), 7.09(1H, d, J=6.9Hz), 7.37(1H, dd, J=6.9Hz, J=7.6Hz), 7.71(1H, d, J=7.6Hz) |

- cont'd -

Table 18 (cont'd)

| Example No. | Compound | NMR ($CDCl_3$, TMS) δ: |
|---|---|---|
| 134 | 9-amino-8-methyl-1-thia-2,3-dihydro-1H-cyclopenta-[b]quinoline | 2.95(3H, s), 3.47(3H, s), 4.75(2H, brs, $NH_2$), 7.10 (1H, d, J=7.0Hz), 7.36(1H, dd, J=7.0Hz, J=7.6Hz), 7.72 (1H, d, J=7.6Hz) |
| 135 | 9-amino-6-fluoro-1-thia-1,2,3,4-tetra-hydroacridine | 2.22-2.31(2H, m), 3.06-3.13 (4H, m), 4.89(2H, brs, $NH_2$), 7.14-7.21(1H, m), 7.51(1H, dd, J=10.3Hz, J=2.4Hz), 7.64(1H, dd, J=9.2Hz, J=5.6Hz) |
| 136 | 9-amino-6-fluoro-3-thia-1,2,3,4-tetra-hydroacridine | 2.90-2.96(2H, m), 3.03-3.08 (2H, m), 3.99(2H, s), 4.71 (2H, brs, $NH_2$), 7.16-7.23 (1H, m), 7.51(1H, dd, J=10.2Hz, J=2.6Hz), 7.70(1H, dd, J=9.2Hz, J=5.8Hz) |

- cont'd -

Table 18 (cont'd)

| Example No. | Compound | NMR ($CDCl_3$, TMS) δ: |
|---|---|---|
| 137 | 9-amino-6-tri-fluoromethyl-1-thia-1,2,3,4-tetrahydro-acridine | 2.25-2.34(2H, m), 3.10-3.18 (4H, m), 4.91(2H, brs, $NH_2$), 7.55-7.59(1H, m), 7.76(1H, d, J=8.9Hz), 8.19-8.20 (1H, m) |
| 138 | 9-amino-7-fluoro-1-thia-1,2,3,4-tetra-hydroacridine | 2.23-2.32(2H, m), 3.10-3.15 (4H, m), 4.73(2H, brs, $NH_2$), 7.23-7.36(2H, m), 7.87(1H, dd, J=9.1Hz, J=5.4Hz) |
| 139 | 9-amino-7-fluoro-3-thia-1,2,3,4-tetra-hydroacridine | 2.94(2H, t, J=5.9Hz), 3.06 (2H, t, J=5.9Hz), 3.99(2H, s), 4.57(2H, brs, $NH_2$), 7.28-7.42(2H, m), 7.89(1H, dd, J=9.2Hz, J=5.6Hz) |
| 140 | 9-amino-6-tri-fluoromethyl-1-thia-1,2,3,4-hydroacridine | 2.25-2.34(2H, m), 3.10-3.18 (4H, m), 4.91(2H, brs, $NH_2$), 7.55-7.59(1H, m), 7.74-7.77 (1H, m), 8.19(1H, s) |

- cont'd -

Table 18 (cont'd)

| Example No. | Compound | NMR ($CDCl_3$, TMS) δ: |
|---|---|---|
| 141 | 9-amino-6-trifluoromethyl-3-thia-1,2,3,4-tetrahydroacridine | 2.93-3.10(4H, m), 4.02(2H, s), 4.77(2H, brs, $NH_2$), 7.56-7.60(1H, m), 7.82(1H, d, J=8.6Hz), 8.20(1H, s) |
| 142 | 9-amino-6,7-difluoro-2-thia-1,2,3,4-tetrahydroacridine | 2.87-3.26(4H, m), 3.72(2H, s), 5.93(2H, brs, $NH_2$), 7.48-7.57(1H, m), 7.99-8.04 (1H, m) |
| 143 | 9-amino-6,7-difluoro-1-thia-1,2,3,4-tetra-acridine | 2.22-2.32(2H, m), 3.05-3.14 (4H, m), 4.74(2H, brs, $NH_2$), 7.33-7.40(1H, m), 7.58-7.66 (1H, m) |
| 144 | 9-amino-6,7-difluoro-3-thia-1,2,3,4-tetrahydroacridine | 2.87-3.06(4H, m), 3.87(2H, s), 5.99(2H, brs, $NH_2$), 7.44-7.52(1H, m), 8.02-8.10(1H, m) |

- cont'd -

Table 18 (cont'd)

| Example No. | Compound | NMR ($CDCl_3$, TMS) δ: |
|---|---|---|
| 145 | 9-amino-5-fluoro-1-thia-1,2,3,4-tetrahydroacridine | 2.23-2.32(2H, m), 3.11-3.19 (4H, m), 4.88(2H, brs, $NH_2$), 7.23-7.44(3H, m) |
| 146 | 9-amino-5-fluoro-3-thia-1,2,3,4-tetrahydroacridine | 2.92-3.08(4H, m), 4.07(2H, s), 4.72(2H, brs, $NH_2$), 7.26-7.49(3H, m) |
| 147 | 9-amino-7-chloro-1-thia-1,2,3,4-tetrahydroacridine | 2.23-2.32(2H, m), 3.07-3.15 (4H, m), 4.80(2H, brs, $NH_2$), 7.49(1H, dd, J=2.3Hz, J=8.9Hz), 7.63(1H, d, J=2.3Hz), 7.81(1H, d, J=8.9Hz) |
| 148 | 9-amino-7-chloro-3-thia-1,2,3,4-tetrahydroacridine | 2.91-3.08(4H, m), 3.99(2H, s), 4.64(2H, brs, $NH_2$), 7.54 (1H, dd, J=2.3Hz, J=8.9Hz), 7.68(1H, d, J=2.3Hz), 7.82 (1H, d, J=8.9Hz) |

- cont'd -

Table 18 (cont'd)

| Example No. | Compound | NMR ($CDCl_3$, TMS) δ: |
|---|---|---|
| 149 | 9-amino-8-chloro-3-thia-1,2,3,4-tetrahydroacridine | 2.80-2.84(2H, m), 3.04-3.08 (2H, m), 3.96(2H, s), 5.91 (2H, brs, $NH_2$), 7.33-7.45(2H, m), 7.75-7.78 (1H, m) |
| 150 | 9-amino-7-methyl-1-thia-1,2,3,4-tetrahydroacridine | 2.22-2.32(2H, m), 2.51(3H, s), 3.07-3.13(4H, m), 4.80 (2H, brs, $NH_2$), 7.38-7.41 (2H, m), 7.76-7.80(1H, m) |
| 151 | 9-amino-7-methyl-3-thia-1,2,3,4-tetrahydroacridine | 2.53(3H, s), 2.91-3.07(4H, m), 4.00(2H, s), 4.62(2H, brs, $NH_2$), 7.43-7.48(2H, m), 7.77-7.80(1H, m) |
| 152 | 9-amino-8-methyl-1-thia-1,2,3,4-tetrahydroacridine | 2.21-2.30(2H, m), 2.96(3H, s), 3.04-3.11(4H, m), 5.23 (2H, brs, $NH_2$), 7.08-7.10 (1H, m), 7.34-7.40(1H, m), 7.69-7.72(1H, m) |

- cont'd -

Table 18 (cont'd)

| Example No. | Compound | NMR ($CDCl_3$, TMS) δ: |
|---|---|---|
| 153 | 9-amino-8-methyl-3-thia-1,2,3,4-tetrahydroacridine | 2.82-2.86(2H, m), 2.97(3H, s), 3.03-3.07(2H, m), 3.97 (2H, s), 5.00(2H, brs, $NH_2$), 7.09-7.12(1H, m), 7.38-7.44 (1H, m), 7.69-7.72(1H, m) |
| 154 | 9-amino-6,7-difluoro-1-thia-1,2,3,4-tetrahydroacridine | 2.22-2.32(2H, m), 3.05-3.14 (4H, m), 4.74(2H, brs, $NH_2$), 7.33-7.40(1H, m), 7.58-7.66 (1H, m) |
| 155 | 9-amino-6,7-difluoro-3-thia-1,2,3,4-tetrahydroacridine | 2.87-3.06(4H, m), 3.87(2H, s), 5.99(2H, brs, $NH_2$), 7.44-7.52(1H, m), 8.02-8.10 (1H, m) |

Table 19

| Example No. | Compound | NMR ($CDCl_3$, TMS) δ: |
|---|---|---|
| 158 | 9-amino-1,2,3,4-tetrahydro-2,4-methanoacridine | 1.63(2H, dd, J=2.6Hz, J=6.6Hz), 2.54-2.62(2H, m), 2.86(2H, d, J=2.6Hz), 2.93-2.97(1H, m), 3.37-3.43(1H, m), 4.56(2H, brs), 7.37-7.43 (1H, m), 7.55-7.61(1H, m), 7.70-7.73(1H, m), 7.90-7.94 (1H, m). |
| 159 | 9-amino-8-fluoro-1,2,3,4-tetrahydro-2,4-methano-acridine | 1.58-1.65(2H, m), 2.53-2.60 (2H, m), 2.78(2H, d, J=3.0Hz), 2.92-2.99(1H, m), 3.32-3.38(1H, m), 5.16(2H, brs), 6.94-7.03(1H, m), 7.39-7.47(1H, m), 7.65-7.69(1H, m) |

Table 20

| Example No. | Compound | NMR ($CD_3OD$, TMS) δ: |
|---|---|---|
| 160 | 9-amino-6-fluoro-1,4-dihydro-1,4-ethanoacridine | 1.52-1.74(4H, m), 4.04-4.14 (2H, m), 6.54-6.61(2H, m), 7.13-7.20(1H, m), 7.46-7.51 (1H, m), 7.81(1H, dd, J=9.2Hz, J=5.9Hz) |
| 161 | 9-amino-8-chloro-1,4-dihydro-1,4-ethanoacridine | 1.52-1.70(4H, m), 4.03-4.13 (2H, m), 6.54-6.60(2H, m), 7.33-7.43(2H, m), 7.77(1H, dd, J=8.3Hz, J=1.7Hz) |

Table 21

| Example No. | Compound | NMR ($CDCl_3$, TMS) δ: |
|---|---|---|
| 162 | 9-amino-1,2,3,4-tetrahydro-1,3-ethanoacridine | 1.70-2.10(6H, m), 2.66-2.94 (2H, m), 3.20-3.30(2H, m), 4.86(2H, brs), 7.29-7.37 (1H, m), 7.51-7.57(1H, m), 7.69-7.72(1H, m), 7.82-7.85 (1H, m) |
| 163 | 9-amino-8-fluoro-1,2,3,4-tetrahydro-1,3-ethanoacridine | 1.73-2.11(6H, m), 2.66-2.86 (2H, m), 3.14-3.30(2H, m), 5.31(2H, brs), 6.90-6.98 (1H, m), 7.36-7.44(1H, m), 7.61(1H, dd, J=8.6Hz, J=1.3Hz) |
| 164 | 9-amino-6-fluoro-1,2,3,4-tetrahydro-1,3-ethanoacridine | 1.73-2.07(6H, m), 2.68-2.96 (2H, m), 3.19-3.31(2H, m), 4.68(2H, brs), 7.10-7.18 (1H, m), 7.48(1H, dd, J=10.6Hz, J=2.6Hz), 7.68 (1H, dd, J=9.2Hz, J=5.6Hz) |

Table 21 (cont'd)

| Example No. | Compound | NMR ($CDCl_3$, TMS) δ: |
|---|---|---|
| 165 | 9-amino-6,8-difluoro-1,2,3,4-tetrahydro-1,3-ethanoacridine | 1.65-2.10(6H, m), 2.60-2.83 (2H, m), 3.11-3.27(2H, m), 5.30(2H, brs), 6.73-6.83 (1H, m), 7.24-7.27(1H, m) |
| 166 | 9-amino-8-methyl-1,2,3,4-tetrahydro-1,3-ethanoacridine | 1.71-2.10(6H, m), 2.63-2.88 (2H, m), 2.95(3H, s), 3.15-3.29(2H, m), 4.95(2H, brs), 7.05(1H, d, J=6.9Hz), 7.35 (1H, dd, J=8.5Hz, J=6.9Hz), 7.68(1H, d, J=8.5Hz) |

Table 22

| Example No. | Compound | NMR ($CDCl_3$, TMS) δ: |
|---|---|---|
| 167 | 9-amino-6-fluoro-1,2,3,4-tetrahydro-2,4-ethanoacridine | 1.86-2.11(6H, m), 2.33-2.38 (1H, m), 2.76-2.84(2H, m), 3.27-3.30(1H, m), 3.52(2H, brs), 7.09-7.16 (1H, m), 7.33-7.49(1H, m), 7.75(1H, dd, J=9.2Hz, J=5.9Hz) |
| 168 | 11-amino-4-fluoro-7,8,9,10-tetrahydro-6H-cyclohepta[b]-quinoline | 1.69-1.75(4H, m), 1.88-1.90(2H, m), 2.83-2.87 (2H, m), 3.08-3.12(2H, m), 4.87(2H, brs), 7.23-7.32 (2H, m), 7.77-7.81(1H, m) |

Example 174

Preparation of 9-amino-1,2,3,4-tetrahydro-2,4-methanoacridine

To nitrobenzene (10 ml) were added anhydrous zinc chloride (1.24 g), 2-aminobenzonitrile (0.53 g) and bicyclo[3.1.1]hepta-2-one (0.5 g), and the resulting mixture was heated one hour at 110°C - 120°C. After cooling, tetrahydrofuran (5 ml) and water (5 ml) were added to the reaction mixture. The mixture was made alkaline by adding 10% aqueous sodium hydroxide solution thereto, followed by three times extractions with ethyl acetate. The organic layer was washed with a 10% aqueous sodium chloride solution and made acidic by adding 10% hydrochloric acid, followed by three times extractions with toluene. The aqueous layer was made alkaline by 10% aqueous sodium hydroxide solution, followed by three times extractions with ethyl acetate. The organic layer was washed two times with 10% aqueous sodium chloride solution, dried over anhydrous magnesium sulfate. Evaporation of ethyl acetate gave crystalline product, which was washed with small amounts of ethyl acetate and ethylether to give 9-amino-1,2,3,4-tetrahydro-2,4-methanoacridine, of which NMR spectrom data was same as that of the desired compound in Example 158.

Experimental Example

Determination of inhibitory activity against acetylcholinesterase

According to the experimental example mentioned below, it is demonstrated that the compounds of the present invention exhibit strong inhibitory activity against acetylcholinesterase and a selective action to the central nervous system and therefore are useful for treatment of senile dementia.

The inhibitory activity against acetylchalinesterase of the compounds provided with the present invention was determined by the photometric methods described in Ellman et al., Biochemical Pharmacology, Vol. 7, p. 88 (1961) and Motohatsu FUJIWARA and Shoji SHIBATA, "Pharmacological basic experimental method", p. 137, Kyorin Shoin.

The results are shown in Table 23.

Table 23

| Example No. | Acetylcholinesterase Inhibition (reative ratio*) |
|---|---|
| 2 | 2.53 |
| 118 | 3.13 |
| 94 | 2.22 |
| 36 | 1.15 |
| 6 | 1.75 |
| 173 | 1.94 |
| 38 | 2.04 |
| 79 | 1.33 |
| 14 | 1.27 |
| 156 | 2.50 |
| 157 | 3.60 |
| 81 | 3.00 |
| 80 | 1.43 |
| 169 | 5.24 |
| 171 | 1.56 |
| 170 | 2.20 |

- cont'd -

Table 23 (cont'd)

| Example No. | Acetylcholinesterase Inhibition (reative ratio*) |
| --- | --- |
| 149 | 4.12 |
| 112 | 1.30 |
| Prior art compound, 9-amino-1,2,3,4-tetrahydroacridine hydrochloride (Tacrine) | 1.00 |

$$*: \text{Reative ratio} = \frac{IC_{50}\ \text{(molar) of Tacrine}}{IC_{50}\ \text{(molar) of Test compounds}}$$

$IC_{50}$ (molar): Concentration of drug which inhibits 50% level of control.

## Claims

1. A compound of the formula,

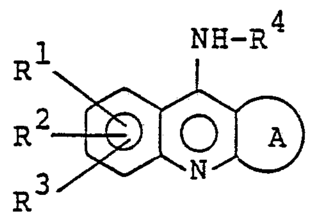

(I)

and pharmaceutically acceptable acid addition salts thereof wherein $R^1$, $R^2$, $R^3$, $R^4$, and

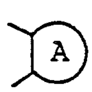

are defined as the following (1) or (2);

(1) 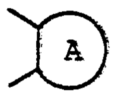 is 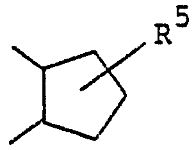 , 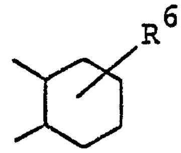 , or

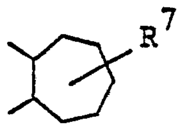

wherein $R^5$, $R^6$ and $R^7$ are each independently hydrogen or lower alkyl; $R^1$ is lower alkyl, halogen, trifluoromethyl, nitro, amino, hydroxy, lower alkylamino, lower alkanoylamino, lower alkylthio, lower alkoxy, or lower alkoxymethyl; $R^2$ and $R^3$ are each independently hydrogen, halogen, lower alkyl, lower alkoxy, lower cycloalkyl, phenyl, or substituted phenyl; and $R^4$ is hydrogen; provided that when $R^1$ is lower alkyl, lower alkoxy, chlorine, bromine, or iodine and $R^2$ and $R^3$ are each hydrogen,

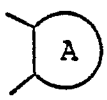 is 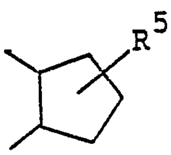 or 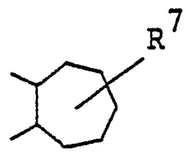

wherein $R^5$ and $R^7$ are each independently lower alkyl;

(2) 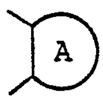 is 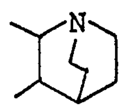 , 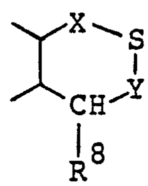 ,

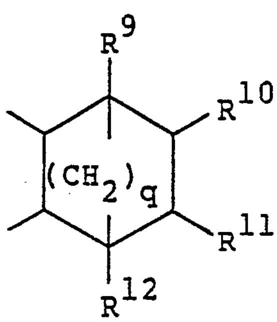 , 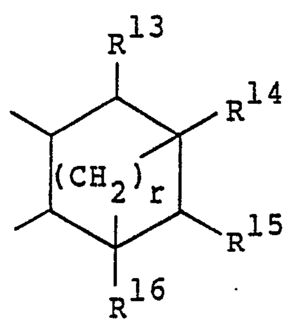 , 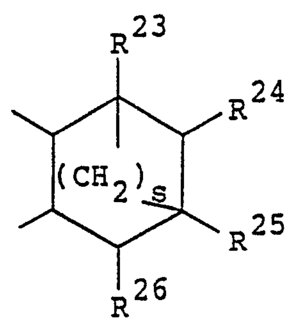 ,

or 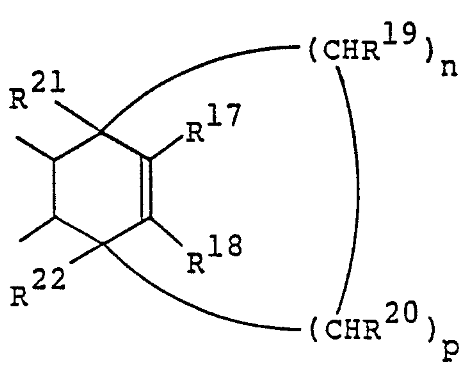

wherein n, q, r, and s are each independently 1 or 2, p is 0 or 1, X and Y are each independently a bond, alkylene, or substituted alkylene, and $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, and $R^{26}$ are each independently hydrogen or lower alkyl, provided that the total of carbon atoms of X and Y is an integer 1 to 3; $R^1$, $R^2$, and $R^3$ are each independently hydrogen, halogen, trifluoromethyl, nitro, amino, hydroxy, lower alkyl, lower alkoxy, lower alkylamino, lower alkanoylamino, lower cycloalkyl, lower alkylthio, lower alkoxymethyl, phenyl, or substituted phenyl; and $R^4$ is hydrogen, lower alkyl, aralkyl, or diaralkyl.

2. The compound according to claim 1, wherein

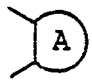 is 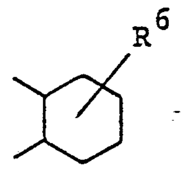

(wherein $R^6$ is hydrogen) or lower alkyl; $R^1$ is fluorine; and $R^2$ and $R^3$ are each independently hydrogen, halogen, lower alkyl, or lower alkoxy.

3. The compound according to claim 1, wherein

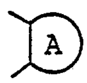 is 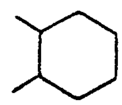 ,

$R^1$ is fluorine, and $R^2$ and $R^2$ are each independently hydrogen or fluorine.

4. The compound according to claim 1, wherein

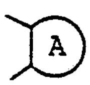 is 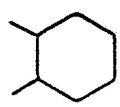 ,

$R^1$ is fluorine, and $R^2$ and $R^3$ are hydrogen.

5. The compound according to claim 1, wherein

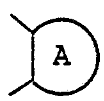 is 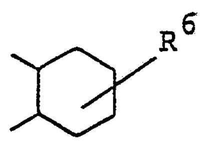 ,

wherein $R^6$ is hydrogen or lower alkyl; $R^1$ is trifluoromethyl; and $R^2$ and $R^3$ are each independently hydrogen, halogen, lower alkyl, or lower alkoxy.

6. The compound according to claim 1, wherein

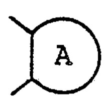 is 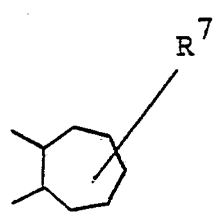

($R^7$ is hydrogen), $R^1$ is fluorine, $R^2$ is hydrogen or fluorine, and $R^3$ is hydrogen.

7. The compound according to claim 1,

wherein 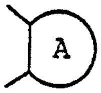 is 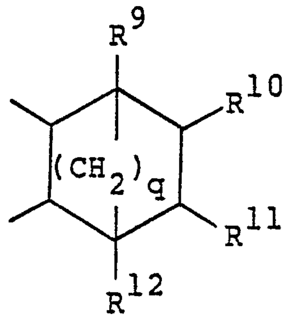 , 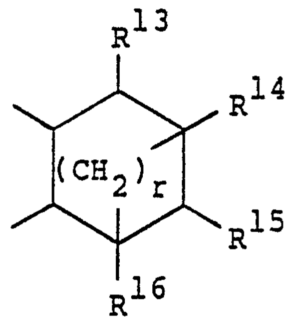 , or

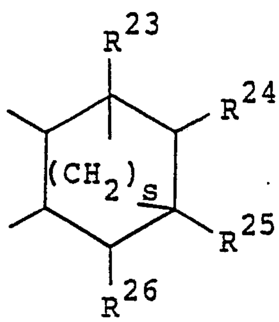

wherein q, r, and s are each independently 1 or 2, and $R^9$, $R^{10}$, $R^{11}$, $R^{12}$, $R^{13}$, $R^{14}$, $R^{15}$, $R^{16}$, $R^{23}$, $R^{24}$, $R^{25}$, and $R^{26}$ are each independently hydrogen or lower alkyl; and $R^1$, $R^2$, and $R^3$ are each independently hydrogen, halogen, lower alkyl, lower alkoxy, or trifluoromethyl.

8. The compound according to claim 1, wherein

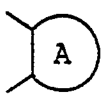 is 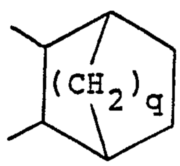 , 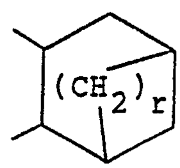 , or

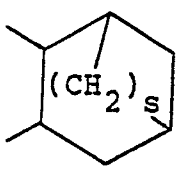

wherein q, r, and s are each independently 1 or 2, and $R^1$, $R^2$, and $R^3$ are each independently hydrogen, halogen, trifluoromethyl, lower alkyl, or lower alkoxy; and $R^4$ is hydrogen.

9. The compound according to claim 1, wherein

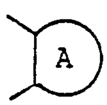 is 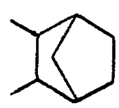 ,

$R^1$, $R^2$, and $R^3$ are each independently hydrogen, halogen, trifluoromethyl, lower alkyl, or lower alkoxy, and $R^4$ is hydrogen.

10. The compound according to claim 1, wherein

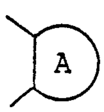 is 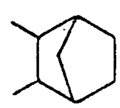 ,

$R^1$, $R^2$, and $R^3$ are each independently hydrogen or halogen, and $R^4$ is hydrogen.

11. The compound according to claim 1, wherein

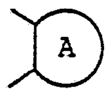 is 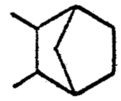 ,

$R^1$, $R^2$, and $R^3$ are each independently hydrogen or fluorine, and $R^4$ is hydrogen.

12. The compound according to claim 1, wherein

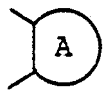 is 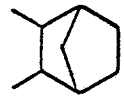 ,

$R^1$ and $R^2$ are each fluorine, and $R^3$ and $R^4$ are each hydrogen.

13. The compound according to claim 1,

wherein 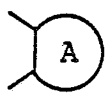 is 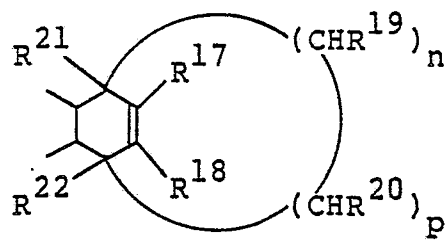

wherein $R^{17}$, $R^{18}$, $R^{19}$, $R^{20}$, $R^{21}$, and $R^{22}$ are each independently hydrogen or lower alkyl, and n is 1 or 2, and p is 0 or 1; and $R^1$, $R^2$, and $R^3$ are each independently hydrogen, halogen, trifluoromethyl, lower alkyl, or lower alkoxy.

14. The compound according to claim 1, wherein

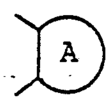 is 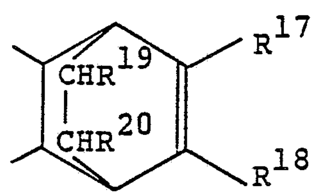

wherein $R^{17}$, $R^{18}$, $R^{19}$, and $R^{20}$ are each independently hydrogen or lower alkyl; $R^1$, $R^2$, and $R^3$ are each independently hydrogen, halogen, trifluoromethyl, lower alkyl, or lower alkoxy; and $R^4$ is hydrogen.

15. The compound according to claim 1, wherein

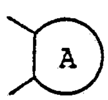 is 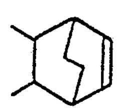 ,

$R^1$, $R^2$, and $R^3$ are each independently hydrogen, halogen, trifluoromethyl, lower alkyl, or lower alkoxy, and $R^4$ is hydrogen.

16. The compound according to claim 1, wherein

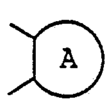 is 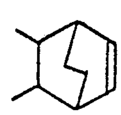 ,

$R^1$, $R^2$, and $R^3$ are each independently hydrogen or halogen, and $R^4$ is hydrogen.

17. The compound according to claim 1, wherein

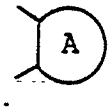 is 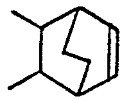 ,

$R^1$, $R^2$, and $R^3$ are each hydrogen, and $R^4$ is hydrogen.

18. The compound according to claim 1, wherein

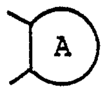 is 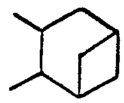 ,

$R^1$, $R^2$, and $R^3$ are each independently hydrogen, halogen, trifluoromethyl, lower alkyl, or lower alkoxy, and $R^4$ is hydrogen.

19. The compound according to claim 1, wherein

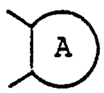 is 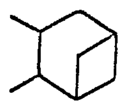 ,

$R^1$, $R^2$, and $R^3$ are each independently hydrogen or halogen, and $R^4$ is hydrogen.

20. The compound according to claim 1, wherein

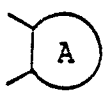 is 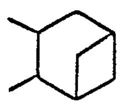 ,

$R^1$, $R^2$, $R^3$ and $R^4$ are each hydrogen.

21. The compound according to claim 1, wherein

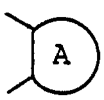 is 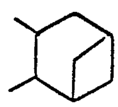 ,

$R^1$ is fluorine, and $R^2$, $R^3$, and $R^4$ are each hydrogen.

22. A pharmaceutical composition, which comprises at least one of the compounds of any of claims 1 to 21 as an active ingredient, and at least one pharmaceutically acceptable carrier or diluent.

23. The pharmaceutical composition according to claim 22, which is for treatment of senile dementia.

24. Use of the compounds of any of clamis 1 to 21 for the preparation of pharmaceutical compositions according to claim 22.

25. Use according to claim 24 for the preparation of pharmaceutical composition according to claim 23.

26. A process for producing a compound of the formula,

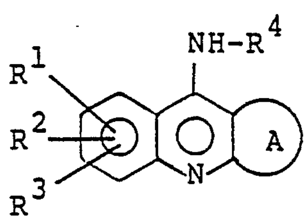

(I)

according to claim 1 and pharmaceutically acceptable acid addition saltsthereof

which comprises

(a) reacting a compound (II) of the formula,

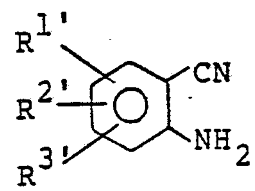

(II)

wherein $R^{1'}$ is lower alkyl, lower alkoxy, chlorine, bromine, or iodine and $R^{2'}$ and $R^{3'}$ are each hydrogen, with a compound (III) or (V) of the formula,

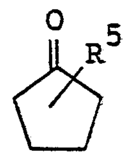

or

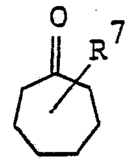

(III) (V)

wherein $R^5$ and $R^7$ are each lower alkyl, in the presence of Lewis acid or dehydrating-condensing agent, and if necessary subjecting the thus obtained product to a salt-forming reaction;

(b) reacting a compound (II) of the formula,

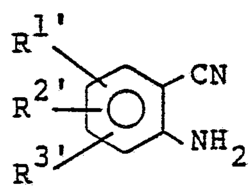

(II)

wherein $R^{1'}$ is lower alkyl, halogen, trifluoromethyl, nitro, hydroxy, lower alkanoylamino, lower alkylthio, lower alkoxy, or lower alkoxymethyl; $R^{2'}$ and $R^{3'}$ are each independently hydrogen, halogen, lower alkyl, lower alkoxy, lower cycloalkyl, phenyl, or substituted phenyl; provided that the case is excluded where $R^1$ is lower alkyl, lower alkoxy, chlorine, bromine, or iodine and $R^2$ and $R^3$ are each hydrogen, with a compound (III), (IV) or (V) of the formula,

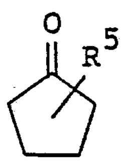

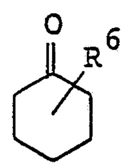

or

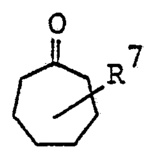

(III) (IV) (V)

wherein $R^5$, $R^6$ and $R^7$ are each independently hydrogen or lower alkyl, in the presence of Lewis acid or dehydrating-condensing agent, and if necessary subjecting the thus obtained product to a salt-forming reaction and/or to reduction of the nitro group,and if necessary to acylation or alkylation of the amino group formed therefrom; or

(c) reacting a compound (II) of the formula,

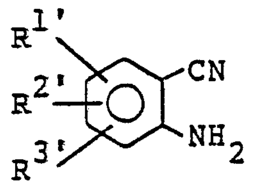

(II)

$R^{1'}$, $^{2'}$ and $R^{3'}$ are each independently hydrogen, halogen, trifluoromethyl, nitro, hydroxy, lower alkyl, lower alkoxy, lower alkanoylamino, lower cycloalkyl, lower alkylthio, lower alkoxymethyl, phenyl, or substituted phenyl, with a compound (VI), (VII), (VIII), (IX), (X) or (XI) of the formula,

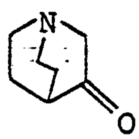

(VI)

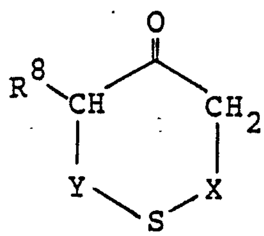

(VII)

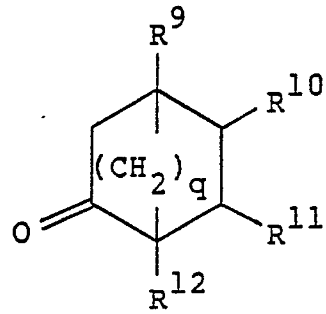

(VIII)

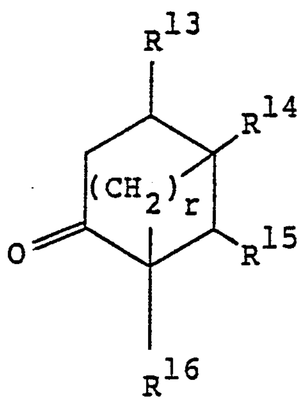

(IX)

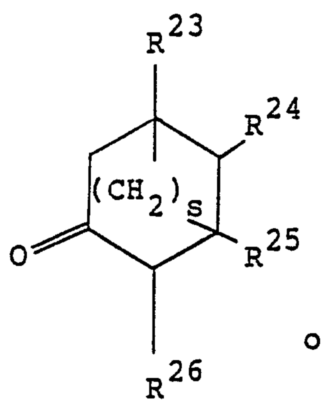

(X)

or

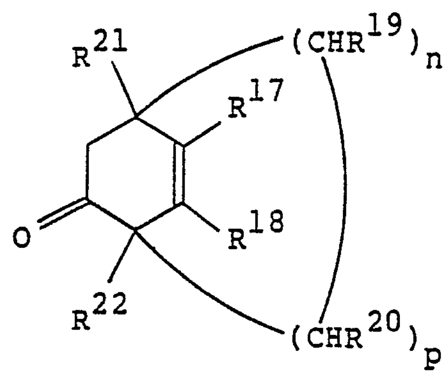

(XI)

wherein n, q, r, and s are each independently 1 or 2, p is 0 or 1, X and Y are each independently a bond, alkylene, or substituted alkylene, and $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, and $R^{26}$ are each independently hydrogen or lower alkyl, provided that the total of carbon atoms of X and Y is an integer 1 to 3, in the presence of Lewis acid or dehydrating-condensing agent, and if necessary subjecting the thus obtained product to a salt-forming reaction; and/or to the reduction of the nitro group and if necessary to an acylation or alkylation of the amino group formed therefrom; and/or to alkylation, aralkylation or diaralkylation of the amino group of $R^4$.

European Patent Office

# EUROPEAN SEARCH REPORT

Application Number

EP 87 11 6008

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 41, no. 19, 10th October 1947, columns 6255a-6256a, Columbus, Ohio, US; V.E. PETROW "Some amino derivatives of dihydro-beta-quinindene and tetrahydroacridine"; & JOURNAL OF THE CHEMICAL SOCIETY 1947, 634-637 * column 6255h, i * | 1 | C 07 D 219/10<br>C 07 D 221/16<br>C 07 D 221/22<br>C 07 D 471/18<br>C 07 D 495/04<br>A 61 K 31/645<br>A 61 K 31/47 //<br>(C 07 D 471/18<br>C 07 D 221:00<br>C 07 D 221:00<br>C 07 D 221:00 )<br>(C 07 D 495/04<br>C 07 D 335:00<br>C 07 D 221:00 )<br>(C 07 D 495/04<br>C 07 D 333:00<br>C 07 D 221:00 ) |
| A | CHEMICAL ABSTRACTS, vol. 89, no. 5, 31th July 1978, page 599, column 1, abstract no. 43073f, Columbus, Ohio, US; K.C. JOSHI et al.: "Possible psychopharmacological agents. Part I. Synthesis of some fluorine containing 9/10-substituted cyclopenta(b)quinolines and cyclohexa(b)quinolines"; & INDIAN JOURNAL OF CHEMISTRY, Sect. B, 1978, 16(2), 156-158 | 1-5,22 | |
| A,D | US-A-3 232 945 (M.V. SIGAL JR. et al.) * column 2, lines 37-59, column 3, lines 15-19; examples 4, 5, 20-27, 31, 32, 52-58 * | 1,22 | **TECHNICAL FIELDS SEARCHED (Int. Cl.4)**<br>C 07 D 219/00<br>C 07 D 221/00<br>C 07 D 471/00<br>C 07 D 495/00<br>A 61 K 31/00 |
| A | CHEMICAL ABSTRACTS, vol. 81, no. 19, 11th November 1974, page 515, column 2, abstract no. 120411d, Columbus, Ohio, US; M.E. KONSHIN et al.: "2,3-Polymethylenequinolines. XIX. Synthesis and biological activity of 4-amino-2,3-polymethylene quinolines" & KHIM.-FARM.ZH 1974, 8(7), 17-19 -/- | 1,22 | |

The present search report has been drawn up for all claims

| Place of search | Date of completion of the search | Examiner |
|---|---|---|
| BERLIN | 10-02-1988 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 87 11 6008

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 100, no. 1, 2nd January 1984, page 146, column 1, abstract no. 1672r, Columbus, Ohio, US; J. BAJGAR et al.: "Protective effect of 9-amino-7-methoxy-1,2,3,4-tetrahydroacridine against inhibition of acetylcholinesterase by O-ethyl S-(2-dimethylaminoethyl) methylphosphonothioate in vivo"; & ARCH. TOXICOL. 1983, 54(2), 163-166 --- | 1,22-25 | |
| A | US-A-3 541 066 (M. WOLF) * whole document * ----- | 1,22,23 | |

TECHNICAL FIELDS SEARCHED (Int. Cl.4)

The present search report has been drawn up for all claims

| Place of search | Date of completion of the search | Examiner |
|---|---|---|
| BERLIN | 10-02-1988 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)